(19) Europäisches Patentamt / European Patent Office / Office européen des brevets

(11) **EP 4 177 269 A1**

(12) **EUROPEAN PATENT APPLICATION**

(43) Date of publication:
**10.05.2023 Bulletin 2023/19**

(21) Application number: **22205436.3**

(22) Date of filing: **04.11.2022**

(51) International Patent Classification (IPC):
*C07K 16/24* (2006.01)     *C07K 16/28* (2006.01)
*C07K 16/32* (2006.01)     *C07K 16/40* (2006.01)

(52) Cooperative Patent Classification (CPC):
**C07K 16/2887; C07K 16/244; C07K 16/248;**
**C07K 16/32; C07K 16/40;** C07K 2317/567;
C07K 2317/92

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority:  **05.11.2021   JP 2021181139**

(71) Applicant: **SYSMEX CORPORATION**
**Kobe-shi**
**Hyogo 651-0073 (JP)**

(72) Inventors:
• **EGASHIRA, Yuriko**
  **Kobe-shi, Hyogo, 651-0073 (JP)**
• **FUKUNAGA, Atsushi**
  **Kobe-shi, Hyogo, 651-0073 (JP)**

(74) Representative: **De Clercq & Partners**
**Edgard Gevaertdreef 10a**
**9830 Sint-Martens-Latem (BE)**

Remarks:
The complete document including Reference Table(s) and the Sequence Listing(s) can be downloaded from the EPO website

(54) **METHOD FOR PRODUCING ANTIBODY**

(57)     Disclosed is a method for producing an antibody, comprising culturing in a presence of a polyanionic compound an animal cell into which a gene encoding an antibody has been introduced whereby the animal cell produces the antibody, wherein the polyanionic compound is at least one selected from the group consisting of an anionic polysaccharide and an anionic polyamino acid, and the antibody is an antibody in which at least 3 amino acid residues of framework region 3 (FR3) are substituted each independently with an arginine residue or a lysine residue.

EP 4 177 269 A1

**Description**

FIELD OF THE INVENTION

The present invention relates to a method for producing an antibody.

BACKGROUND

There has been known a technique for controlling affinity of an antibody for an antigen by modifying the amino acid sequence of a framework region (FR) while maintaining the amino acid sequence of the complementarity determining regions (CDRs) of the antibody. FR is a region other than CDRs, present in each variable region of the light chain and heavy chain of the antibody.

For example, U.S. Patent Application Publication No. 2018/0179298 describes a method for controlling affinity for an antigen by changing at least 3 amino acid residues in FR3 of an antibody to charged amino acid residues. The antibody obtained by the method described in U.S. Patent Application Publication No. 2018/0179298 is expected to be used in medicine, an in vitro diagnostic agent, a reagent, and the like.

In order to obtain an antibody in which at least 3 amino acid residues of FR3 are substituted with arginine residues or lysine residues, the present inventors cultured an animal cell into which a gene encoding the antibody had been introduced by a conventional method. However, the amount of the obtained antibody was not sufficiently satisfactory. The present inventors aimed to provide a means to increase yield of the antibody.

SUMMARY OF THE INVENTION

Generally, in the production of an antibody by a protein expression system using an animal cell, the yield of antibody increases when the scale of cell culture is expanded. However, production cost is high. The present inventors have studied a means to increase the yield of antibody regardless of expansion of the scale of cell culture. As a result, the present inventors have found that the yield of antibody is increased by culturing the animal cell into which a gene encoding the antibody is introduced in the presence of a polyanionic compound, thereby completing the present invention.

The present invention provides a method for producing an antibody, comprising culturing in a presence of a polyanionic compound an animal cell into which a gene encoding an antibody has been introduced whereby the animal cell produces the antibody, wherein the polyanionic compound is at least one selected from the group consisting of an anionic polysaccharide and an anionic polyamino acid, and the antibody is an antibody comprising in a framework region 3 (FR3) at least 3 amino acids which are arginine or lysine. In particular embodiments, the antibody is an antibody in which at least 3 amino acid residues of a framework region 3 (FR3) are substituted each independently with an arginine residue or a lysine residue.

In a certain embodiment, the anionic polysaccharide is a sulfated polysaccharide or alginate.

In a certain embodiment, the sulfated polysaccharide is at least one selected from the group consisting of dextran sulfate , a salt thereof, glycosaminoglycan, and proteoglycan comprising a sulfate group.

In a certain embodiment, the glycosaminoglycan is at least one selected from the group consisting of heparin, heparan sulfate, chondroitin sulfate, dermatan sulfate, keratan sulfate, pentosan sulfate, and salts thereof.

In a certain embodiment, the proteoglycan comprising a sulfate group is a protein to which at least one selected from the group consisting of heparin, heparan sulfate, chondroitin sulfate, dermatan sulfate, keratan sulfate, and salts thereof is covalently bonded.

In a certain embodiment, the anionic polyamino acid is at least one selected from the group consisting of polyglutamic acid, polyaspartic acid, and salts thereof.

In a certain embodiment, the animal cell is cultured in a medium comprising the polyanionic compound at a concentration of 0.05 mg/mL or more and 20 mg/mL or less.

In a certain embodiment, the antibody is IgG.

In a certain embodiment, the at least 3 amino acid residues of the FR3 comprise residues at at least three positions selected from the group consisting of positions 63, 65, 67, 70 and 72 of a light chain defined by Chothia method.

In a certain embodiment, the antibody is an antibody in which amino acid residues at positions 63, 65 and 67 comprise or are substituted each independently with an arginine residue or a lysine residue.

In a certain embodiment, the antibody is an antibody in which amino acid residues at positions 63, 65, 67 and 70 comprise or are substituted each independently with an arginine residue or a lysine residue.

In a certain embodiment, the antibody is an antibody in which amino acid residues at positions 63, 65, 67, 70 and 72 comprise or are substituted each independently with an arginine residue or a lysine residue.

In a certain embodiment, the antibody is an antibody in which at least 3 amino acid residues of a framework region 3 (FR3) have been substituted each independently with an arginine residue or a lysine residue and which antibody has improved affinity for an antigen as compared with an antibody before substitution of the amino acid residues.

According to the present invention, in the production of antibody by a protein expression system using an animal cell, the yield of the antibody can be increased.

BRIEF DESCRIPTION OF THE DRAWINGS

Fig. 1 is a graph showing viability (%) when a CHO-S cell line stably expressing rituximab was cultured in a medium to which glucose, sucrose or trehalose had been added;

Fig. 2 is a graph showing viability (%) when a CHO-S cell line stably expressing R3 variant of rituximab was cultured in a medium to which glucose, sucrose or trehalose had been added;

Fig. 3 is a graph showing viability (%) when a CHO-S cell line stably expressing R5 variant of rituximab was cultured in a medium to which glucose, sucrose or trehalose had been added;

Fig. 4 is a graph showing viability (%) when a CHO-S cell line stably expressing rituximab was cultured in a medium to which dextran sulfate sodium, sodium chondroitin sulfate or heparin sodium had been added;

Fig. 5 is a graph showing viability (%) when a CHO-S cell line stably expressing R3 variant of rituximab was cultured in a medium to which dextran sulfate sodium, sodium chondroitin sulfate or heparin sodium had been added;

Fig. 6 is a graph showing viability (%) when a CHO-S cell line stably expressing R5 variant of rituximab was cultured in a medium to which dextran sulfate sodium, sodium chondroitin sulfate or heparin sodium had been added;

Fig. 7 is a graph showing antibody concentration (mg/L) in a supernatant when a CHO-S cell line stably expressing rituximab was cultured in a medium to which glucose, sucrose or trehalose had been added;

Fig. 8 is a graph showing antibody concentration (mg/L) in a supernatant when a CHO-S cell line stably expressing R3 variant of rituximab was cultured in a medium to which glucose, sucrose or trehalose had been added;

Fig. 9 is a graph showing antibody concentration (mg/L) in a supernatant when a CHO-S cell line stably expressing R5 variant of rituximab was cultured in a medium to which glucose, sucrose or trehalose had been added;

Fig. 10 is a graph showing antibody concentration (mg/L) in a supernatant when a CHO-S cell line stably expressing rituximab was cultured in a medium to which dextran sulfate sodium, sodium chondroitin sulfate or heparin sodium had been added;

Fig. 11 is a graph showing antibody concentration (mg/L) in a supernatant when a CHO-S cell line stably expressing R3 variant of rituximab was cultured in a medium to which dextran sulfate sodium, sodium chondroitin sulfate or heparin sodium had been added;

Fig. 12 is a graph showing antibody concentration (mg/L) in a supernatant when a CHO-S cell line stably expressing R5 variant of rituximab was cultured in a medium to which dextran sulfate sodium, sodium chondroitin sulfate or heparin sodium had been added;

Fig. 13 is a graph showing viability (%) when a CHO-S cell line stably expressing rituximab was cultured in a medium containing dextran sulfate sodium at a concentration of 0.1, 1 or 10 mg/mL;

Fig. 14 is a graph showing viability (%) when a CHO-S cell line stably expressing R5 variant of rituximab was cultured in a medium containing dextran sulfate sodium at a concentration of 0.1, 1 or 10 mg/mL;

Fig. 15 is a graph showing antibody concentration (mg/L) in a supernatant when a CHO-S cell line stably expressing rituximab was cultured in a medium containing dextran sulfate sodium at a concentration of 0.1, 1 or 10 mg/mL;

Fig. 16 is a graph showing antibody concentration (mg/L) in a supernatant when a CHO-S cell line stably expressing R5 variant of rituximab was cultured in a medium containing dextran sulfate sodium at a concentration of 0.1, 1 or 10 mg/mL;

Fig. 17 is a graph showing viability (%) when a CHO-S cell line stably expressing rituximab was cultured in a medium containing dextran sulfate sodium with a molecular weight of 5,000 or 50,000;

Fig. 18 is a graph showing viability (%) when a CHO-S cell line stably expressing R5 variant of rituximab was cultured in a medium containing dextran sulfate sodium with a molecular weight of 5,000 or 50,000;

Fig. 19 is a graph showing antibody concentration (mg/L) in a supernatant when a CHO-S cell line stably expressing rituximab was cultured in a medium containing dextran sulfate sodium with a molecular weight of 5,000 or 50,000;

Fig. 20 is a graph showing antibody concentration (mg/L) in a supernatant when a CHO-S cell line stably expressing R5 variant of rituximab was cultured in a medium containing dextran sulfate sodium with a molecular weight of 5,000 or 50,000;

Fig. 21 is a graph showing viability (%) when a CHO-S cell line stably expressing R3 variant of rituximab was cultured in a medium containing dextran sulfate sodium with a molecular weight of 5,000, 50,000 or 500,000;

Fig. 22 is a graph showing antibody concentration (mg/L) in a supernatant when a CHO-S cell line stably expressing R3 variant of rituximab was cultured in a medium containing dextran sulfate sodium with a molecular weight of 5,000, 50,000 or 500,000;

Fig. 23 is a graph showing viability (%) when a CHO-S cell line stably expressing rituximab was cultured in a medium containing heparin sodium at a concentration of 0.1, 1 or 10 mg/mL;

Fig. 24 is a graph showing viability (%) when a CHO-S cell line stably expressing R5 variant of rituximab was cultured in a medium containing heparin sodium at a concentration of 0.1, 1 or 10 mg/mL;

Fig. 25 is a graph showing antibody concentration (mg/L) in a supernatant when a CHO-S cell line stably expressing rituximab was cultured in a medium containing heparin sodium at a concentration of 0.1, 1 or 10 mg/mL;

Fig. 26 is a graph showing antibody concentration (mg/L) in a supernatant when a CHO-S cell line stably expressing R5 variant of rituximab was cultured in a medium containing heparin sodium at a concentration of 0.1, 1 or 10 mg/mL;

Fig. 27 is a graph showing viability (%) when a CHO-S cell line stably expressing rituximab was cultured in a medium containing sodium polyaspartate or sodium polyglutamate;

Fig. 28 is a graph showing viability (%) when a CHO-S cell line stably expressing R3 variant of rituximab was cultured in a medium containing sodium polyaspartate or sodium polyglutamate;

Fig. 29 is a graph showing viability (%) when a CHO-S cell line stably expressing R5 variant of rituximab was cultured in a medium containing sodium polyaspartate or sodium polyglutamate;

Fig. 30 is a graph showing antibody concentration (mg/L) in a supernatant when a CHO-S cell line stably expressing rituximab was cultured in a medium containing sodium polyaspartate or sodium polyglutamate;

Fig. 31 is a graph showing antibody concentration (mg/L) in a supernatant when a CHO-S cell line stably expressing R3 variant of rituximab was cultured in a medium containing sodium polyaspartate or sodium polyglutamate;

Fig. 32 is a graph showing antibody concentration (mg/L) in a supernatant when a CHO-S cell line stably expressing R5 variant of rituximab was cultured in a medium containing sodium polyaspartate or sodium polyglutamate;

Fig. 33 is a graph showing viability (%) when a CHO-S cell line stably expressing rituximab was cultured in a medium containing dextran or dextran sulfate sodium;

Fig. 34 is a graph showing viability (%) when a CHO-S cell line stably expressing R3 variant of rituximab was cultured in a medium containing dextran or dextran sulfate sodium;

Fig. 35 is a graph showing viability (%) when a CHO-S cell line stably expressing R5 variant of rituximab was cultured in a medium containing dextran or dextran sulfate sodium;

Fig. 36 is a graph showing antibody concentration (mg/L) in a supernatant when a CHO-S cell line stably expressing rituximab was cultured in a medium containing dextran or dextran sulfate sodium;

Fig. 37 is a graph showing antibody concentration (mg/L) in a supernatant when a CHO-S cell line stably expressing R3 variant of rituximab was cultured in a medium containing dextran or dextran sulfate sodium;

Fig. 38 is a graph showing antibody concentration (mg/L) in a supernatant when a CHO-S cell line stably expressing R5 variant of rituximab was cultured in a medium containing dextran or dextran sulfate sodium;

Fig. 39 is a graph showing viability (%) when a CHO-S cell line stably expressing rituximab was cultured in a medium containing polyproline or sodium alginate;

Fig. 40 is a graph showing viability (%) when a CHO-S cell line stably expressing R3 variant of rituximab was cultured in a medium containing polyproline or sodium alginate;

Fig. 41 is a graph showing antibody concentration (mg/L) in a supernatant when a CHO-S cell line stably expressing rituximab was cultured in a medium containing polyproline or sodium alginate;

Fig. 42 is a graph showing antibody concentration (mg/L) in a supernatant when a CHO-S cell line stably expressing R3 variant of rituximab was cultured in a medium containing polyproline or sodium alginate;

Fig. 43 is a graph showing an affinity of trastuzumab and its variant for an antigen;

Fig. 44 is a graph showing an affinity of an anti-lysozyme antibody and its variant for an antigen;

Fig. 45 is a graph showing an affinity of an anti-interleukin (IL)-6 antibody and its variant for an antigen;

Fig. 46 is a graph showing an affinity of an anti-IL-8 antibody and its variant for an antigen; and

Fig. 47 is a graph showing an affinity of rituximab and its variant for an antigen.

## DETAILED DESCRIPTION OF THE PREFERRED EMBODIMENTS

In the method for producing an antibody of the present invention, in vitro, an animal cell into which a gene encoding an antibody has been introduced is cultured in the presence of a polyanionic compound so that the animal cell produces an antibody. Here, the antibody encoded by the gene introduced into the animal cell and the antibody produced by the animal cell are antibodies in which at least 3 amino acid residues of FR3 comprise (i.e. correspond to) or are substituted with arginine residues or lysine residues (the latter hereinafter, also referred to as "variant").

As used herein, the "antibody" may be any of IgG, IgA, IgM, IgD and IgE, and is preferably IgG. In the case of producing IgG, it is preferable to use an animal cell expression vector in which a gene encoding a full-length light chain and a gene encoding a full-length heavy chain are incorporated as a gene encoding an antibody. The "antibody" may be an antibody fragment. The antibody fragment preferably contains a variable region of a light chain. Examples of such an antibody fragment include Fab, Fab', F(ab')2, Fd, Fd', Fv, scFv, domain antibody (dAb), reduced IgG (rIgG), diabody, triabody, and the like.

An animal cell into which a gene encoding an antibody has been introduced (hereinafter, also referred to as "transgenic cell") is an animal cell into which the gene has been introduced so that an antibody as a protein can be expressed. The transgenic cell may be, for example, an animal cell in which the gene is incorporated into an intracellular genome, or an animal cell holding the gene as an episomal vector. An animal cell into which a gene encoding an antibody is introduced in such a form is called a stable cell line, and can constitutively express an antibody as a protein. Alternatively, the transgenic cell may be an animal cell transiently expressing an antibody as a protein by transfection of the gene. The antibody expressed by the transgenic cell is secreted from the cell into culture supernatant.

An animal cell is a host cell that expresses a protein by gene transfer. Examples of the animal cell include mammalian cells, insect cells, and the like. Among them, a mammalian cell is particularly preferable. The mammalian cell may be a primary cultured cell prepared from a tissue or an established cell line. Preferably, a mammalian cell line is used. Examples of the cell line include CHO cells (including CHO-K1 and CHO-S), HEK293 cells (including HEK293T and HEK293E), BHK cells, COS cells, HeLa cells, VERO cells, 3T3 cells, and the like. The cell line can be obtained from the American Type Culture Collection (ATCC), the European Collection of Cell Cultures (ECACC), and the like. Commercially available cell lines such as Expi293 (trademark) cell and ExpiCHO (trademark) cell may be used.

The gene encoding an antibody may be incorporated into an animal cell expression vector. The type of animal cell expression vector is not particularly limited, and may be, for example, a plasmid vector, a viral vector, or the like. The gene encoding an antibody includes a gene encoding a light chain of the antibody and a gene encoding a heavy chain of the antibody. When the gene encoding an antibody is incorporated into the animal cell expression vector, the gene encoding a light chain and the gene encoding a heavy chain of the antibody may be incorporated into one vector or may be incorporated into two vectors, respectively.

According to the methods of the invention, the gene encoding the antibody is or has been introduced into a host cell. Introduction of the gene encoding an antibody into an animal cell itself can be performed by a known transfection method. The type of transfection method is not particularly limited, and examples thereof include lipofection method, calcium phosphate co-precipitation method, viral vector method, electroporation method, and the like.

Culture in the presence of a polyanionic compound can be performed, for example, by culturing a transgenic cell in a medium to which the polyanionic compound has been added. By culturing the transgenic cell in the presence of a polyanionic compound, yield of the antibody or antibody variant by the cell is increased as compared to the case of culturing in the absence of a polyanionic compound. Details of the mechanism by which the yield is increased are not clear, but it is considered that electrostatic interaction between an antibody or antibody variant secreted from the transgenic cell into the culture supernatant and the polyanionic compound in the medium contributes. However, the present invention is not bound by a specific theory or the like.

Culture in the absence of a polyanionic compound refers to culturing the transgenic cell in a medium substantially free of a polyanionic compound. The medium substantially free of a polyanionic compound refers to a medium in which the amount of polyanionic compound is small even if the polyanionic compound is contained, and the polyanionic compound is not actively added. Examples of the case where a trace amount of the polyanionic compound is contained in a medium include a case where the polyanionic compound is contained in the additive described later.

The polyanionic compound refers to a polymer having an anionic compound having an anionic functional group as a monomer unit and a salt thereof. Examples of the anionic functional group include a sulfuric acid group, a carboxyl group, and the like. The salt of the polyanionic compound is preferably a pharmaceutically acceptable salt, and examples thereof include sodium salts and potassium salts. In the present invention, an anionic polysaccharide and/or an anionic polyamino acid is used as the polyanionic compound. Examples of the anionic polysaccharide include sulfated polysaccharides and alginates. Examples of the anionic polyamino acid include polyglutamic acid, polyaspartic acid, and salts thereof. The polyanionic compound may be one type or two or more types.

Examples of the sulfated polysaccharide include dextran sulfate and salts thereof, glycosaminoglycan, and proteoglycan containing a sulfate group. The dextran sulfate is preferably a pharmaceutically acceptable salt, and examples thereof include sodium salts and potassium salts. Particularly preferred is dextran sulfate sodium.

The alginate is preferably a pharmaceutically acceptable salt of alginic acid, and examples thereof include sodium alginate, potassium alginate, and ammonium alginate. Among them, sodium alginate is particularly preferable.

The glycosaminoglycan refers to an acidic polysaccharide containing an amino sugar. Examples of the glycosaminoglycan include heparin, heparan sulfate, chondroitin sulfate, dermatan sulfate, keratan sulfate, pentosan sulfate, and salts thereof. Chondroitin sulfate has various structures depending on the type of sugar as a constituent and the position of the sulfate group. Examples of the type of chondroitin sulfate include chondroitin sulfate A (chondroitin 4-sulfate), chondroitin sulfate C (chondroitin 6-sulfate), chondroitin sulfate D (chondroitin 6-sulfate having a sulfate group at position 2 of glucuronic acid), chondroitin sulfate E (chondroitin sulfate having sulfate groups at positions 4 and 6 of N-acetylgalactosamine), and the like. Chondroitin sulfate B is the same as dermatan sulfate. In the present invention, any chondroitin sulfate may be used.

The salt of glycosaminoglycan is preferably a pharmaceutically acceptable salt, and examples thereof include sodium salts and potassium salts. Particularly preferred is a sodium salt. In the present invention, it is preferable to use heparin sodium, sodium heparan sulfate, sodium chondroitin sulfate, sodium dermatan sulfate, sodium keratan sulfate, or sodium pentosan sulfate. Among them, heparin sodium and sodium chondroitin sulfate are particularly preferably used.

The proteoglycan refers to a compound in which glycosaminoglycan and a protein are covalently bonded. Examples of the proteoglycan containing a sulfate group include proteins in which any one selected from heparin, heparan sulfate, chondroitin sulfate, dermatan sulfate, keratan sulfate, and salts thereof is covalently bonded. Such proteins are known per se and include, and examples thereof include aggrecan, versican, neurocan, brevican, decorin, biglycan, syndecan, glypican, and the like.

The anionic polyamino acid preferably contains a plurality of acidic amino acids. The acidic amino acid contained in the anionic polyamino acid is any one or both of glutamic acid and aspartic acid. The anionic polyamino acid may include a neutral amino acid and/or a basic amino acid, but when the anionic polyamino acid includes a basic amino acid, the number of residues of the basic amino acid is less than the number of residues of the acidic amino acid. The anionic polyamino acid preferably does not contain a basic amino acid and more preferably consists only of acidic amino acids. The anionic polyamino acid may be any of a polymer composed of L-form amino acids, a polymer composed of D-form amino acids, and a polymer containing both L-form and D-form amino acids. The anionic polyamino acid salt is preferably a pharmaceutically acceptable salt, and examples thereof include sodium salts and potassium salts. Particularly preferred are sodium polyglutamate and sodium polyaspartate.

The average molecular weight of the dextran sulfate sodium may be about 5,000 or more and about 500,000 or less. As used herein, the "average molecular weight" is a weight average molecular weight measured by gel permeation chromatography (GPC). When a commercially available polyanionic compound is used, the molecular weight of the compound may be a value disclosed by a manufacturer or a supplier.

The lower limit of the concentration of the polyanionic compound in the medium is preferably higher than 0.01 mg/mL. The lower limit of the concentration may be, for example, 0.02 mg/mL, 0.03 mg/mL, 0.04 mg/mL, 0.05 mg/mL, 0.06 mg/mL, 0.07 mg/mL, 0.08 mg/mL, 0.09 mg/mL, or 0.1 mg/mL. The upper limit of the concentration of the polyanionic compound in the medium is not particularly limited, but is, for example, 20 mg/mL or less. The upper limit of the concentration may be, for example, 19 mg/mL, 18 mg/mL, 17 mg/mL, 16 mg/mL, 15 mg/mL, 14 mg/mL, 13 mg/mL, 12 mg/mL, 11 mg/mL, or 10 mg/mL. For example, the transgenic cell can be cultured in a medium containing the polyanionic compound at a concentration of 0.05 mg/mL or more and 20 mg/mL or less, and more preferably at a concentration of 0.1 mg/mL or more and 10 mg/mL or less.

The polyanionic compound may be added to the medium in advance or may be added to the medium from the middle of cell culture. When the transgenic cell is a stable cell line, it is preferable to culture in a medium to which a polyanionic compound has been added in advance. When the transgenic cell is a cell transiently expressing an antibody by transfection, it is preferable to add the cell to the medium, for example, 3 to 4 hours after completion of the gene introduction operation. Alternatively, the medium may be replaced with a medium to which the polyanionic compound has been added in advance.

Culture period is not particularly limited, and can be appropriately determined according to, for example, the type of animal cell, the type of protein expression system, a culture method, and the like. When the animal cell is a stable cell line, the culture period may be, for example, 5 days or more and 14 days or less. When the animal cell transiently expresses an antibody or antibody variant by transfection of a gene encoding an antibody or antibody variant, the culture period may be, for example, 24 hours or more and 7 days or less from the completion of the gene introduction operation.

The medium is not particularly limited, and examples thereof include a medium generally used for culturing an animal cell in vitro. Such a medium itself is known, and examples thereof include MEM, DMEM, RPMI-1640, and the like. In addition, a commercially available medium such as CD FortiCHO (trademark) medium or Expi293 (trademark) Expression medium may be used. If necessary, additives such as fetal bovine serum (FBS), L-glutamine, antibiotics, antifungal agents and anti-agglutinating agents may be mixed in the medium. When the transgenic cell is a stable cell line into which a gene expressing a selection marker such as a drug resistance gene has been introduced, it is preferable to add a selection agent to the medium. Selection markers and corresponding selection agents are known.

As culture conditions for an animal cell, known conditions may be adopted depending on the type of cell and the like. For example, a general mammalian cell can be cultured under the condition of 37°C under a 5% $CO_2$ atmosphere. In addition, a culture method may be selected according to the scale of culture. Examples of the culture method include batch culture, fed-batch culture, and continuous culture. The continuous culture includes chemostat culture and perfusion culture. The batch culture is suitable, for example, for small-scale culture at laboratory level. The fed-batch culture and the continuous culture are suitable, for example, for large-scale culture at industrial level.

When the method for producing an antibody of the present invention is carried out by batch culture, a medium containing the transgenic cell and the polyanionic compound is added to a culture vessel or a culture apparatus, and the medium was cultured. Hereinafter, the culture vessel or the culture apparatus containing the transgenic cell cultured in the medium containing the polyanionic compound is also referred to as "culture system". In the batch culture, supply of the medium into the culture system and removal of the cell culture (cell and medium) out of the culture system are not performed during the culture period. When the method for producing an antibody of the present invention is carried out by fed-batch culture, a medium containing the transgenic cell and the polyanionic compound is added to a culture vessel or a culture apparatus, and the medium was cultured. During the culture period, predetermined components (medium component, nutrient source, the additive described above, polyanionic compound, and the like) are continuously or intermittently supplied into the culture system. When the method for producing an antibody of the present invention is carried out by chemostat culture, the medium is continuously supplied to a culture vessel or a culture apparatus to which the medium containing the transgenic cell and the polyanionic compound has been added. At this time, the same amount of cell culture as the supplied medium is continuously taken out of the culture system to maintain the culture system in a steady state. When the method for producing an antibody of the present invention is carried out by perfusion culture, the medium is continuously supplied to a culture vessel or a culture apparatus to which the medium containing the transgenic cell and the polyanionic compound has been added. At this time, the same amount of culture supernatant as the supplied medium is continuously taken out of the culture system to maintain the culture system in a steady state. In the perfusion culture, the cell is not removed.

The culture vessel or the culture apparatus can be appropriately selected according to the type of cell, the culture method, and the like. Examples of the culture vessel include a dish, a microplate, a culture flask (T-flask), a spinner flask, a roller bottle, and the like. Examples of the culture apparatus include a stirred bioreactor, a fluidized bed bioreactor, a fixed bed culture vessel, and the like.

After culturing the transgenic cell in the presence of a polyanionic compound in vitro, the produced antibody or antibody variant is recovered from the culture supernatant or the cell. Since the antibody or antibody variant expressed by the transgenic cell is usually secreted to the outside of the cell, the antibody or antibody variant is present in the culture supernatant. Therefore, by removing the cell from the cell culture and recovering only the culture supernatant, a solution of the antibody or antibody variant can be obtained. When a part of the antibody or antibody variant expressed by the transgenic cell is accumulated in the cell, a solution of the antibody or antibody variant can be obtained by lysing the cell with a solution containing an appropriate solubilizing agent to liberate the antibody or antibody variant. If necessary, the lysate of the cell may be centrifuged to recover the supernatant containing the antibody or antibody variant. The antibody or antibody variant liberated in the liquid may be purified by a known method such as gel filtration chromatography or affinity chromatography.

In the method for producing an antibody of the present invention, the yield of a variant is increased as compared with the yield of an antibody or antibody variant obtained by culture in the absence of a polyanionic compound. On the other hand, the function of the antibody or antibody variant produced in the present invention as an antibody is equivalent to that of the antibody or antibody variant obtained by culture in the absence of a polyanionic compound. For example, the affinity of an antibody or antibody variant obtained by the method for producing an antibody of the present invention for an antigen is the same as that of a antibody or antibody variant obtained by culture in the absence of a polyanionic compound. The affinity for an antigen can be evaluated by, for example, an immunological measurement method such as an ELISA method, a kinetic parameter in an antigen-antibody reaction, or the like. Examples of an index of affinity by immunological measurement include a 50% effective concentration (EC50). Examples of the kinetic parameter include dissociation constant ($K_D$), binding rate constant ($k_{on}$), and dissociation rate constant ($k_{off}$). The kinetic parameter in an antigen-antibody reaction can be obtained by surface plasmon resonance (SPR) technology or the like. Hereinafter, the antibody or antibody variant will be described.

As long as the antibody or antibody variant envisaged for production by the method for producing an antibody of the present invention is an antibody in which at least 3 amino acid residues of FR3 comprise or are substituted with arginine residues or lysine residues, the antigen recognized by the antibody or antibody variant, the animal species from which the antibody or antibody variant is derived, and the like are not particularly limited. Here, FR is a region other than CDRs, present in each variable region of the light chain and heavy chain of the antibody. FR plays a role of a scaffold linking three CDRs and contributes to structural stability of the CDR. Therefore, the amino acid sequence of FR is highly conserved between antibodies of the same species. Each variable region of the heavy chain and light chain has three CDRs, CDR1, CDR2 and CDR3, and four FRs, FR1, FR2, FR3 and FR4. These are arranged in the order of FR1, CDR1, FR2, CDR2, FR3, CDR3 and FR4 from the N-terminal side of the variable region. In particular embodiments the FR3 comprising or substituted with arginine residues or lysine residues is at least the light chain FR3.

The antibody or antibody variant may be, for example, an antibody derived from human, mouse, rat, hamster, rabbit, goat, horse, or chicken. The antibody or antibody variant may be a chimeric antibody, a humanized antibody, a bispecific antibody, or the like.

In particular embodiments, the methods are used for the production of an antibody variant, i.e. an antibody in which at least three amino acid residues of the FR3 in the original antibody have been substituted with arginine or lysine. In the variant, since at least 3 amino acid residues of FR3 are substituted with arginine residues or lysine residues, the affinity of the variant for an antigen is improved as compared with the antibody before the substitution. Hereinafter, an antibody before at least 3 amino acid residues of FR3 are substituted with arginine residues or lysine residues is also referred to as "original antibody". Substituting at least 3 amino acid residues of FR3 of the original antibody with arginine residues or lysine residues is hereinafter also referred to as "modify". The variant may be an antibody described in U.S. Patent Application Publication No. 2018/0179298. U.S. Patent Application Publication No. 2018/0179298 is incorporated herein by reference. In particular embodiments, the original antibody does not comprise an FR3 comprising at least 3 arginine residues or lysine residues. In Particular embodiments, the method for the production of an antibody variant also comprises the steps of providing an original antibody and substituting at least 3 amino acid residues of FR3 of the original antibody with arginine residues or lysine residues so as to obtain an antibody variant or providing a gene of an original antibody and modifying said gene so as to substitute, in the encoded antibody, at least 3 amino acid residues of FR3 of the original antibody with arginine residues or lysine residues so as to obtain a gene encoding an antibody variant. In particular embodiments the at least 3 substituted amino acids of FR3 are 3, 4 or 5 amino acids of FR3.

In the present invention, the antibody or antibody variant is preferably an antibody in which at least 3 amino acid residues of the light chain FR3 comprise or are substituted with arginine residues or lysine residues. In particular embodiments the antibody or antibody variant comprises at least 3, 4 or 5 amino acids in the light chain FR3 which are arginine residues or lysine residues. In one embodiment, in the antibody or antibody variant, at least 3 amino acid residues of the light chain FR3 comprising or substituted with arginine residues or lysine residues include residues at/in at least three positions selected from the group consisting of positions 63, 65, 67, 70 and 72 of a light chain defined by the Chothia method. For example, in the light chain FR3 of the antibody or antibody variant, positions that are arginine residues or lysine residues may be any of the following (1) to (5):

(1) positions 63, 65 and 67 of the light chain defined by the Chothia method;
(2) positions 65, 67 and 70 of the light chain defined by the Chothia method;
(3) positions 63, 65, 67 and 70 of the light chain defined by the Chothia method;
(4) positions 65, 67, 70 and 72 of the light chain defined by the Chothia method; and
(5) positions 63, 65, 67, 70 and 72 of the light chain defined by the Chothia method.

The Chothia method is known as one of methods of numbering the amino acid residues of CDR (hereinafter, also referred to as "numbering method") for defining boundary and length of CDR. When the amino acid residues of CDR are numbered by the numbering method, the amino acid residues of FR are also numbered. The number assigned to the amino acid residue by the numbering method indicates a position of the amino acid residue in the amino acid sequence of the light chain or the heavy chain. In the present specification, boundaries and lengths of CDR and FR3 are defined by Chothia method (see Chothia C. and Lesk AM., Canonical Structures for the Hypervariable Regions of Immunoglobulins., J Mol Biol., vol. 196, pp. 901-917, 1987). In the Chothia method, the light chain FR3 is defined as a region consisting of amino acid residues at positions 57 to 88 of the light chain. Hereinafter, when the position of an amino acid residue in the light chain of an antibody is described, the position of the amino acid residue means a position defined by the Chothia method unless otherwise specified.

In the antibody or antibody variant, amino acid residues other than positions 63, 65, 67, 70 and 72 of the light chain, for example, amino acid residues selected from positions 57 to 62, 74, 76, 77 and 79 to 81 of the light chain may be arginine residues or lysine residues. Here, positions 57 to 62, 74, 76, 77 and 79 to 81 of the light chain are positions of amino acid residues excluding the Vernier zone residue and the unexposed residue from the amino acid sequence of the light chain FR3. The term "Vernier zone residue" is an amino acid residue contributing to structural stability of the CDR among the amino acid sequence of FR. The term "unexposed residue" is an amino acid residue that is folded inside the molecule and is not exposed to the surface.

In the antibody or antibody variant, the amino acid residues present (optionally after being modified from amino acid residues of the original antibody) may be all arginine residues or all lysine residues. Alternatively, a part of the amino acid residues present (optionally after being modified from the amino acid residues of the original antibody) may be arginine residues, and the rest may be lysine residues.

In the present invention, the electrical characteristic of CDR of the antibody or antibody variant is preferably neutral or negatively charged. As used herein, the "electrical characteristic of CDR" is determined by following formula (I):

$$X = [\text{Number of basic amino acid residues in amino acid sequence of CDR contained in one antigen binding site}] - [\text{Number of acidic amino acid residues in amino acid sequence of CDR contained in one antigen binding site}]... \text{ formula (I)}$$

wherein when X is -2 or less, the electrical characteristic of CDR is negatively charged,
when X is -1, 0 or 1, the electrical characteristic of CDR is neutral, and

when X is 2 or more, the electrical characteristic of CDR is positively charged.

The "one antigen binding site" is a site composed of one heavy chain variable region and one light chain variable region, and is a site that binds to an antigen in an antibody. The number of antigen binding sites of an antibody varies depending on class and form of the antibody. For example, when an antibody is IgG or F(ab')2, the antibody has two antigen binding sites. When an antibody is Fab, the antibody has one antigen binding site. The "CDR contained in one antigen binding site" is all CDRs present in one heavy chain variable region and one light chain variable region constituting the one antigen binding site. That is, the CDR contained in one antigen binding site is a total of six CDRs of CDR1, CDR2 and CDR3 in one heavy chain variable region and CDR1, CDR2 and CDR3 in one light chain variable region.

As can be seen from the formula (I), the electrical characteristic of CDR is determined based on the number of acidic amino acid residues and basic amino acid residues in the amino acid sequence of CDR contained in one antigen binding site. When X calculated by the formula (I) is -2 or less, that is, when the number of acidic amino acid residues is larger than the number of basic amino acid residues by 2 or more, in the amino acid sequence of CDR contained in one antigen binding site, the electrical characteristic of CDR is determined to be negatively charged. Also, when X calculated by the formula (I) is -1, 0, or 1, that is, when the difference between the number of acidic amino acid residues and the number of basic amino acid residues is 0 or 1, in the amino acid sequence of CDR contained in one antigen binding site, the electrical characteristic of CDR is determined to be neutral.

In other words, when the total number of acidic amino acid residues contained in the six CDRs of the light chain CDR1, the light chain CDR2, the light chain CDR3, the heavy chain CDR1, the heavy chain CDR2 and the heavy chain CDR3 is larger than the total number of basic amino acid residues by 2 or more, the electrical characteristic of CDR is defined to be negatively charged. In addition, when the difference between the total number of acidic amino acid residues contained in the six CDRs of the light chain CDR1, the light chain CDR2, the light chain CDR3, the heavy chain CDR1, the heavy chain CDR2 and the heavy chain CDR3 and the total number of basic amino acid residues is 0 or 1, the electrical characteristic of CDR is defined to be neutral. As used herein, the acidic amino acid residues are an aspartic acid residue and a glutamic acid residue, and the basic amino acid residues are an arginine residue and a lysine residue. As used herein, a histidine residue is not included in the basic amino acid residues.

In particular embodiments, the methods of the invention comprise the step of providing a gene encoding an antibody or antibody variant and introducing it into an animal cell. A gene encoding an antibody or antibody variant can be acquired using known DNA recombination techniques and other molecular biological techniques, for example, with reference to U.S. Patent Application Publication No. 2018/0179298. First, a polynucleotide encoding the amino acid sequence of the (original) antibody is obtained. For example, when there is a hybridoma that produces an original antibody, RNA extracted from the hybridoma is used to synthesize a polynucleotide encoding the light chain of the original antibody and a polynucleotide encoding the heavy chain, by a reverse transcription reaction and a RACE (Rapid Amplification of cDNA ends) method. These polynucleotides can be used as genes encoding the antibody. Where the antibody does not yet contain 3 arginine or lysine residues in the FR3 region, a variant can be obtained. For instance, from these polynucleotides, a polynucleotide encoding an amino acid sequence in which the light chain FR3 and/or the heavy chain FR3 is modified is prepared. For example, the polynucleotide encoding the light chain of the original antibody as a template is amplified by PCR using a primer for modifying at least 3 amino acid residues of light chain FR3, whereby a polynucleotide encoding the light chain in which FR3 has been modified can be obtained. The obtained polynucleotide and the polynucleotide encoding the heavy chain of the original antibody can be used as genes encoding a variant. If necessary, the gene encoding a variant may be incorporated into an animal cell expression vector.

When there is no hybridoma that produces the (original) antibody, an antibodyproducing hybridoma may be prepared by known methods such as those described in, for example, Kohler and Milstein, Nature, vol.256, pp.495-497, 1975. Alternatively, the polynucleotide encoding the light chain and the polynucleotide encoding the heavy chain of the antibody may be synthesized using RNA obtained from the spleen of an animal such as a mouse immunized with an antigen of interest. Alternatively, an amino acid sequence of an antibody that specifically binds to an antigen of interest or a base sequence of an antibody gene may be acquired from a known database, and gene synthesis may be performed based on these sequences to acquire an antibody gene. Examples of the database include GeneBank, abYsis, IMGT, and the like.

Hereinbelow, the present invention will be described in detail by examples, but the present invention is not limited to these examples.

EXAMPLES

Example 1: Study on Effect of Saccharide and Sulfated Polysaccharide

An animal cell into which a gene encoding rituximab and its variant was introduced was cultured in a medium containing a saccharide or a sulfated polysaccharide, and whether the yield of antibody was increased was studied.

(1) Acquisition of gene of variant of rituximab

In the same manner as in the method described in U.S. Patent Application Publication No. 2018/0179298, a polynucleotide encoding the light chain of rituximab, its R3 variant and its R5 variant was synthesized by PCR using a plasmid DNA containing a gene encoding rituximab (mouse/human chimeric antibody) as a template. In addition, a polynucleotide encoding the heavy chain of rituximab was synthesized. The R3 variant was an antibody in which amino acid residues at positions 63, 65 and 67 of the light chain defined by the Chothia method were substituted with arginine residues. The R5 variant was an antibody in which amino acid residues at positions 63, 65, 67, 70 and 72 of the light chain defined by the Chothia method were substituted with arginine residues. A polynucleotide encoding the light chain of each variant and a polynucleotide encoding the heavy chain of rituximab were inserted into a Freedom (trademark) pCHO 1.0 Expression Vector (Thermo Fisher Scientific Inc.). Thus, an animal cell expression plasmid DNA containing a gene encoding each variant of rituximab was obtained. In addition, an animal cell expression plasmid DNA containing a gene encoding rituximab in which the amino acid residue of the light chain FR3 is not substituted was also obtained.

Based on the base sequence of the gene encoding rituximab, amino acid sequences of a light chain and a heavy chain of the antibody (IgG) were determined. These amino acid sequences were as follows. Also, the amino acid sequences of the light chains of each prepared variant (IgG) are also shown below. The underlined portion indicates a position substituted with an arginine residue.

· Heavy chain of rituximab

QVQLQQPGAELVKPGASVKMSCKASGYTFTSYNMHWVKQTPGRGLEW

IGAIYPGNGDTSYNQKFKGKATLTADKSSSTAYMQLSSLTSEDSAVYYCARSTYY

GGDWYFNVWGAGTTVTVSAASTKGPSVFPLAPSSKSTSGGTAALGCLVKDYFPE

PVTVSWNSGALTSGVHTFPAVLQSSGLYSLSSVVTVPSSSLGTQTYICNVNHKPS

NTKVDKKAEPKSCDKTHTCPPCPAPELLGGPSVFLFPPKPKDTLMISRTPEVTCVV

VDVSHEDPEVKFNWYVDGVEVHNAKTKPREEQYNSTYRVVSVLTVLHQDWLN

GKEYKCKVSNKALPAPIEKTISKAKGQPREPQVYTLPPSRDELTKNQVSLTCLVK

GFYPSDIAVEWESNGQPENNYKTTPPVLDSDGSFFLYSKLTVDKSRWQQGNVFS

CSVMHEALHNHYTQKSLSLSPGK (SEQ ID NO: 1)

· Light chain of rituximab

QIVLSQSPAILSASPGEKVTMTCRASSSVSYIHWFQQKPGSSPKPWIYATS

NLASGVPVRFSGSGSGTSYSLTISRVEAEDAATYYCQQWTSNPPTFGGGTKLEIK

RTVAAPSVFIFPPSDEQLKSGTASVVCLLNNFYPREAKVQWKVDNALQSGNSQES

VTEQDSKDSTYSLSSTLTLSKADYEKHKVYACEVTHQGLSSPVTKSFNRGEC

(SEQ ID NO: 2)

· Light chain of R3 variant of rituximab

QIVLSQSPAILSASPGEKVTMTCRASSSVSYIHWFQQKPGSSPKPWIYATS

NLASGVPVRFRGRGRGTSYSLTISRVEAEDAATYYCQQWTSNPPTFGGGTKLEIK

RTVAAPSVFIFPPSDEQLKSGTASVVCLLNNFYPREAKVQWKVDNALQSGNSQES

VTEQDSKDSTYSLSSTLTLSKADYEKHKVYACEVTHQGLSSPVTKSFNRGEC

(SEQ ID NO: 3)

· Light chain of R5 variant of rituximab

QIVLSQSPAILSASPGEKVTMTCRASSSVSYIHWFQQKPGSSPKPWIYATS

NLASGVPVRFRGRGRGTRYRLTISRVEAEDAATYYCQQWTSNPPTFGGGTKLEIK

RTVAAPSVFIFPPSDEQLKSGTASVVCLLNNFYPREAKVQWKVDNALQSGNSQES

VTEQDSKDSTYSLSSTLTLSKADYEKHKVYACEVTHQGLSSPVTKSFNRGEC

(SEQ ID NO: 4)

(2) Introduction of gene encoding antibody into animal cell

[Reagents]

CHO-S Cells (Thermo Fisher Scientific Inc.)
CD FortiCHO (trademark) medium (Thermo Fisher Scientific Inc.)
Anti-aggregation agent (Thermo Fisher Scientific Inc.)
OptiPRO (trademark) SFM (Thermo Fisher Scientific Inc.)
FreeStyle (trademark) MAX reagent (Thermo Fisher Scientific Inc.)
L-Glutamine (Thermo Fisher Scientific Inc.)
Puromycin dihydrochloride (Thermo Fisher Scientific Inc.)
Methotrexate hydrate (Sigma-Aldrich)

CHO-S cells were proliferated by shaking culture (130 rpm) in a CD FortiCHO (trademark) medium containing 8 mM L-glutamine under 8% $CO_2$ atmosphere at 37°C and 85% humidity. 30 mL of cell culture ($1.0 \times 10^6$ cells/mL) was prepared according to the number of samples. Plasmid DNAs encoding each of rituximab, R3 variant and R5 variant were cleaved with restriction enzyme Pvu I and purified by an ethanol precipitation method to obtain linearized plasmid DNA. A DNA solution having the following composition was prepared using linearized plasmid DNA. Also, a transfection reagent having the following composition was prepared, and the transfection reagent was left for 5 minutes.

[DNA Solution]

| Plasmid DNA solution | Amount ($\mu$L) corresponding to 50 $\mu$g |
|---|---|
| OptiPRO (trademark) SFM | Appropriate amount (mL) |
| Total | 1.5 mL |

[Transfection reagent]

| FreeStyle (trademark) MAX reagent | 80 $\mu$L |
|---|---|
| OptiPRO (trademark) SFM | 1420 $\mu$L |
| Total | 1.5 mL |

The prepared DNA solution and transfection reagent were mixed. The mixture was allowed to stand for 10 minutes. The resulting mixture (3 mL) was added to the cell culture (30 mL). The mixture was shake-cultured (150 rpm) at 37°C in a 5% $CO_2$ atmosphere. After 48 hours, puromycin and methotrexate were added to each culture, and selection of cells into which the gene had been introduced was performed. The medium was replaced and passaged until viability and cell proliferation capability were restored, thereby obtaining CHO-S cells stably expressing rituximab and each variant (hereinafter referred to as "stable cell line"). Cultures of these stable cell lines were cryopreserved by a conventional method.

(3) Culture of stable cell lines

Each cryopreserved stable cell line was thawed and cultured in a CD FortiCHO (trademark) medium containing 1/100 amount of an anti-aggregation agent and 8 mM L-glutamine, and passaging was performed multiple times. A test medium was prepared by adding a saccharide or a sulfated polysaccharide to a CD FortiCHO (trademark) medium containing 8 mM L-glutamine. As a saccharide, D(+) glucose (FUJIFILM Wako Pure Chemical Corporation), sucrose (KISHIDA CHEMICAL CO.,LTD.), or trehalose dihydrate (FUJIFILM Wako Pure Chemical Corporation) was added so as to have a concentration of 50 mM. As a sulfated polysaccharide, sodium chondroitin sulfate C (FUJIFILM Wako Pure Chemical Corporation), dextran sulfate sodium (molecular weight: 5000) (Merck KGaA), or heparin sodium (FUJIFILM Wako Pure Chemical Corporation) was added so as to have a concentration of 1 mg/mL. Each passaged stable cell line was centrifuged at 800 rpm for 5 minutes to completely remove the medium. Each recovered stable cell line was seeded on each test medium at a concentration of $3.0 \times 10^5$ cells/mL, followed by culturing for 14 days. For comparison, each stable cell line was cultured for 14 days in a medium to which a saccharide and a sulfated polysaccharide were not added.

(4) Evaluation of survival and antibody production of stable cell lines

A part of the cell culture was collected as a specimen at the start of culture (Day 0) and on Days 3, 7, 10 and 14 from the start in each test medium. A part of the specimen was used to count living cells and dead cells. In addition, the specimen was centrifuged, and antibody concentration in the supernatant was measured. Cell counting was performed using a hemocytometer after staining the cells with trypan blue. For each stable cell line, viability was calculated from the number of living cells and the number of dead cells. The antibody concentration in the supernatant was measured by sandwich ELISA method. Specific operations were as follows.

A solution of an anti-human IgG Fc antibody (Bethyl Laboratories, Inc.) diluted to 2 $\mu$g/mL with a KPL coating solution (SeraCare Life Sciences, Inc.) was added to each well of a 96-well plate (Corning Inc.), and the plate was incubated at 4°C overnight. After removing the solution from each well, 1% BSA/PBS was added, and the plate was incubated at room temperature for 3 hours. The solution was removed from each well, and then the plate was washed three times with 0.1% Tween (registered trademark) 20-containing PBS (PBST). A culture supernatant was added to each well, and the plate was incubated at room temperature for 1 hour. The solution was removed from each well, and then the plate washed three times with PBST. A solution of an HRP-labeled anti-human IgG Fc antibody (Bethyl Laboratories, Inc.) diluted to 0.3 $\mu$g/mL with 1% BSA/PBS was added to each well, and the plate was incubated at room temperature for 1 hour. The solution was removed from each well, and then the plate washed three times with PBST. Chromogenic reaction was performed using KPL ABTS (registered trademark) Peroxidase Substrate System (SeraCare Life Sciences, Inc.), and the chromogenic reaction was stopped using KPL ABTS (registered trademark) Peroxidase Stop Solution (SeraCare Life Sciences, Inc.). Absorbance at 405 nm was measured using a SpectraMax (registered trademark) 190 microplate reader (Molecular Devices, LLC). The concentration of the antibody in each supernatant was obtained using a standard curve of a reference antibody.

(5) Results

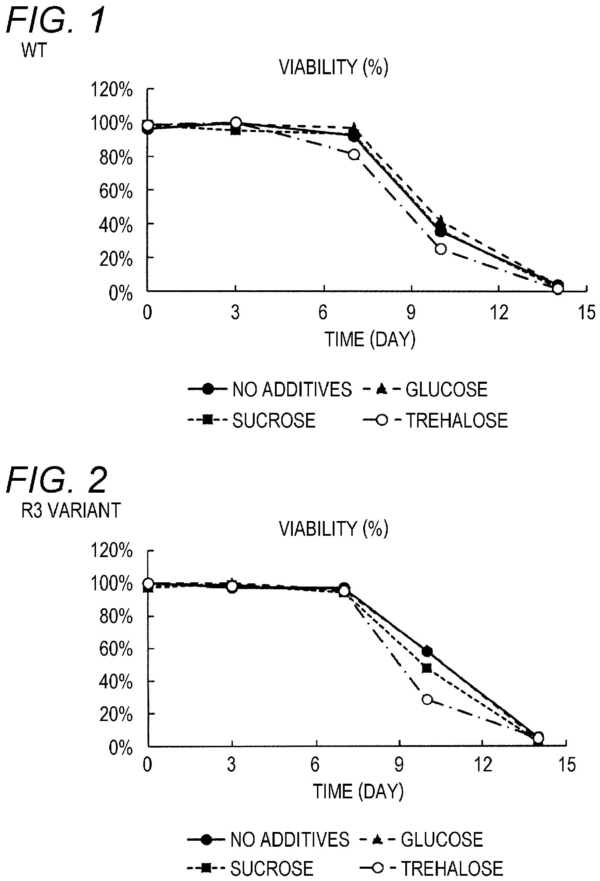

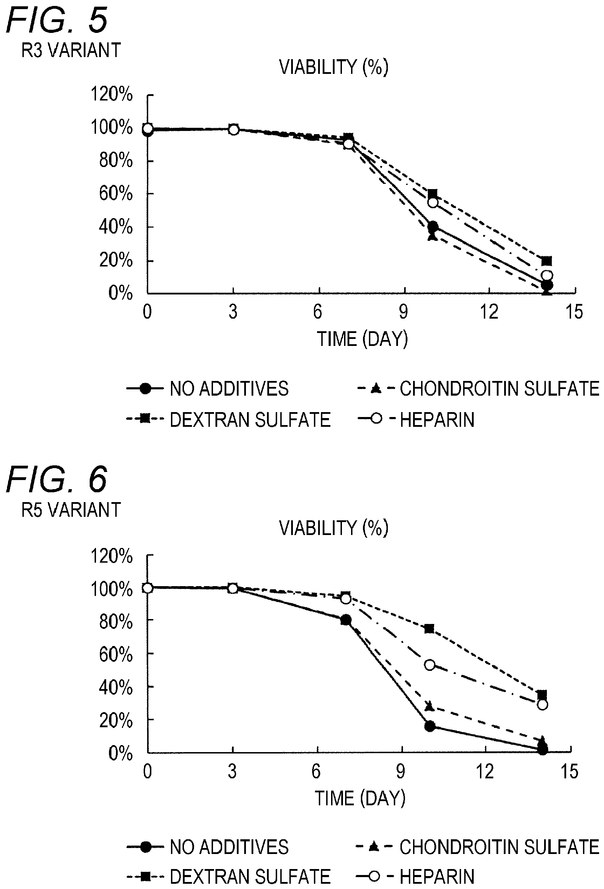

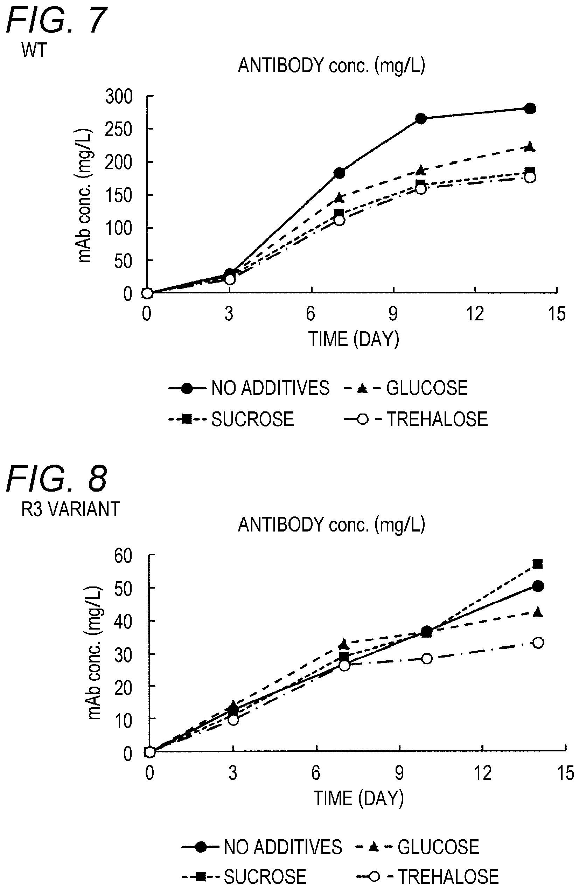

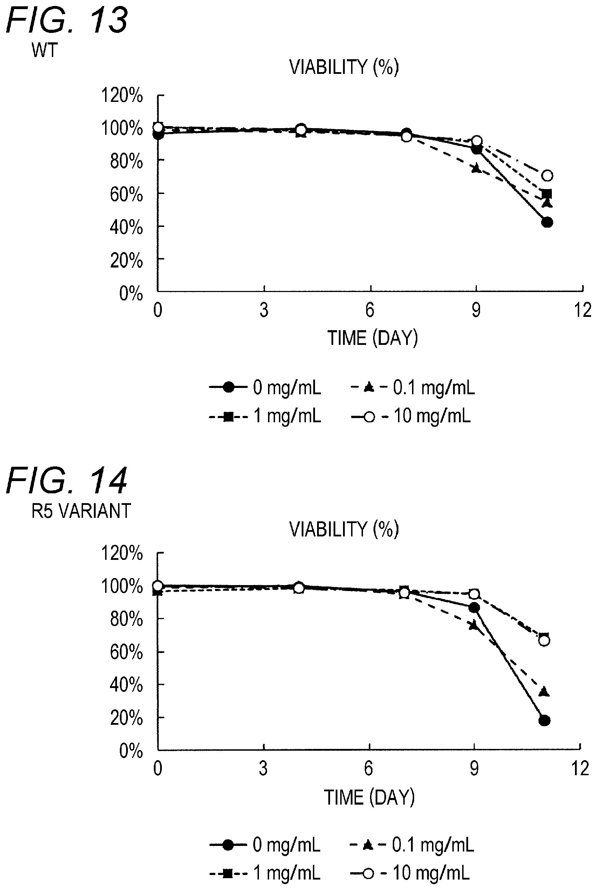

The viability (%) of each stable cell line cultured in a medium to which a saccharide or a sulfated polysaccharide has been added is shown in Figs. 1 to 6. The antibody yield of each stable cell line cultured in a medium to which a saccharide or a sulfated polysaccharide has been added is shown in Figs. 7 to 12 as the antibody concentration (mg/L) in the supernatant. In the figures, "WT" refers to rituximab in which the amino acid residue of the light chain FR3 is not substituted (the same applies to Fig. 13 and subsequent drawings).

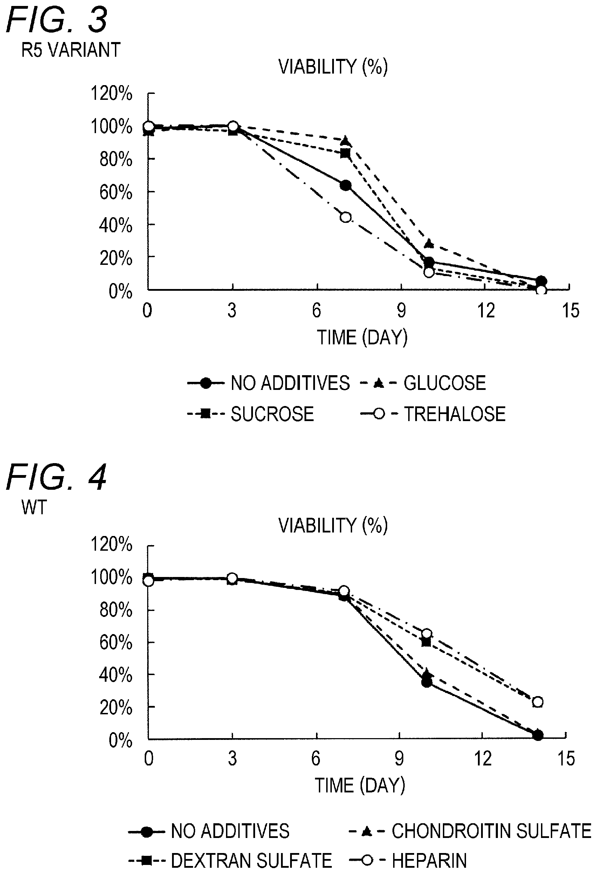

As shown in Figs. 1 to 3, no significant change was observed in the viability of the stable cell lines of rituximab and each variant even when any saccharide had been added. As shown in Figs. 4 to 6, when dextran sulfate sodium and heparin sodium had been added, the viability of each stable cell line on Day 10 and Day 14 tended to be slightly higher as compared to the case where chondroitin sulfate had been added and the case where nothing had been added.

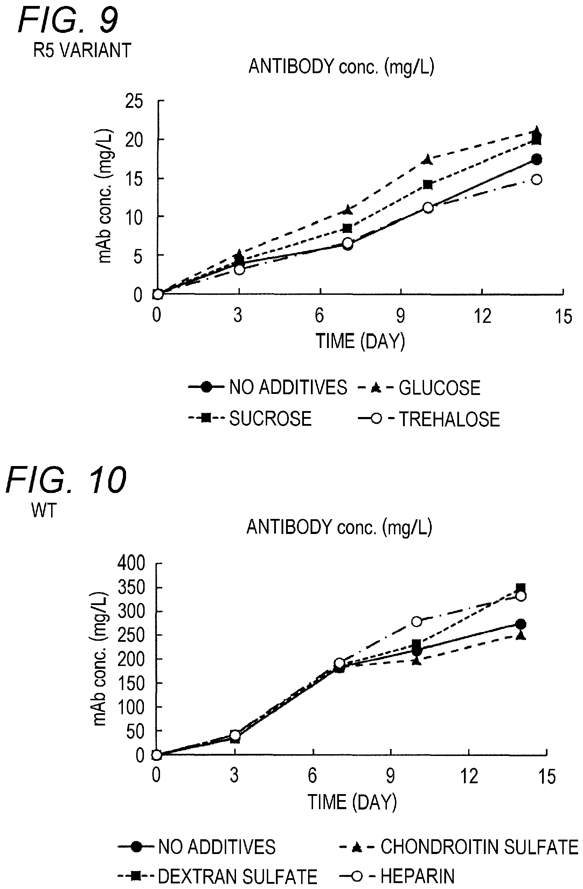

As shown in Fig. 7, regardless of which saccharide had been added, yield of rituximab was lower than that in the case where nothing had been added. As shown in Fig. 8, yield of R3 variant on Day 14 was slightly higher in the case where sucrose had been added than that in the case where nothing had been added. As shown in Fig. 9, yield of R5 variant on Days 7, 10 and 14 were slightly higher in the case where sucrose or glucose had been added than that in the case where saccharide had not been added. From these results, it was suggested that the addition of saccharide may slightly improve the yield of rituximab and each variant. However, the increase in the yield of variant due to the addition of saccharide was not sufficiently satisfactory.

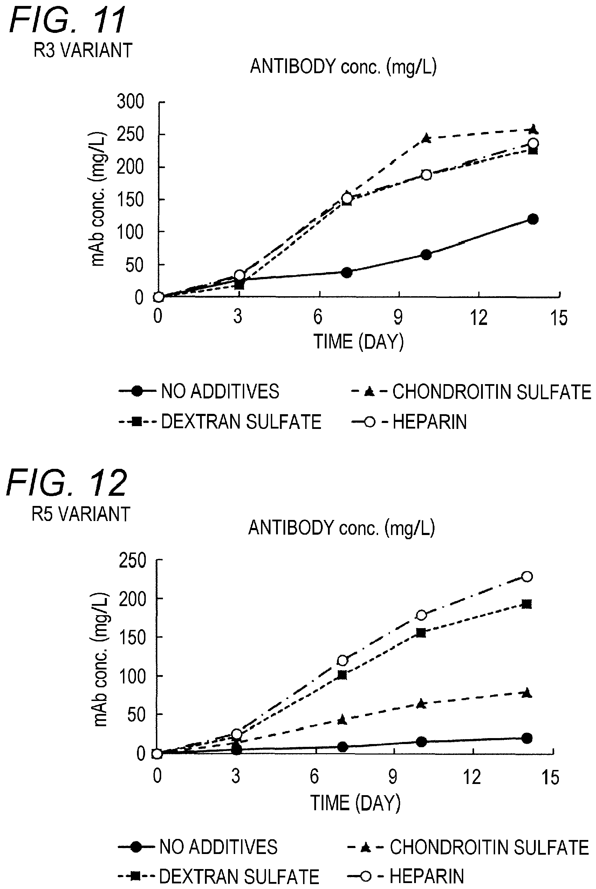

As shown in Fig. 10, the yield of rituximab on Day 14 was slightly higher in the case where dextran sulfate sodium or heparin sodium had been added than in the case where sodium chondroitin sulfate had been added and in the case where nothing had been added. On the other hand, as shown in Figs. 11 and 12, the yields of R3 and R5 variants on Days 7, 10 and 14 were remarkably increased even when any sulfated polysaccharide had been added as compared to that in the case where nothing had been added. Specifically, the yield of R3 variant on Day 14 was about twice the yield when nothing had been added by the addition of sulfated polysaccharide. In addition, the yield of R5 variant on Day 14 was 3.7 times the yield when nothing had been added by the addition of sodium chondroitin sulfate. The yield of R5 variant on Day 14 was about 10 times the yield when nothing had been added by the addition of dextran sulfate sodium and heparin sodium. From these results, it was suggested that the addition of sulfated polysaccharide markedly improves the yields of R3 and R5 variants of rituximab.

Example 2: Study on Concentration and Molecular Weight of Dextran Sulfate Sodium

Each of the stable cell lines of rituximab and R5 variant prepared in Example 1 was cultured in a medium containing dextran sulfate sodium at various concentrations or molecular weights, and whether the yield of antibody was increased was studied.

(1) Culture of stable cell lines

Frozen stocks of each of the stable cell lines of rituximab and R5 variant were thawed, cultured, and passaged in the same manner as in Example 1. In order to study the molecular weight, a test medium was prepared by adding dextran sulfate sodium (Merck KGaA) with a molecular weight of 5,000 or dextran sulfate sodium (FUJIFILM Wako Pure Chemical Corporation) with a molecular weight of 50,000 to a CD FortiCHO (trademark) medium containing 8 mM L-glutamine so as to have a concentration of 1 mg/mL. In addition, in order to study the concentration, a test medium was prepared by adding dextran sulfate sodium (Merck KGaA) with a molecular weight of 5,000 to a CD FortiCHO (trademark) medium containing 8 mM L-glutamine so as to have a concentration of 0.1, 1 or 10 mg/mL. In the same manner as in Example 1, each stable cell line was seeded on each test medium, followed by culturing for 11 or 12 days. For comparison, each stable cell line was cultured for 11 or 12 days in a medium to which dextran sulfate sodium had not been added.

(2) Evaluation of survival and antibody production of stable cell lines

For the experiment of the test medium containing dextran sulfate sodium at different concentrations, a part of the cell culture was collected as a specimen at the start of culture (Day 0) and on Days 4, 7, 9 and 11 from the start. For the experiment of the test medium containing dextran sulfate sodium with different molecular weights, a part of the cell culture was collected as a specimen at the start of culture (Day 0) and on Days 5, 7, 9 and 12 from the start. In the same manner as in Example 1, the cell viability was calculated, and the antibody concentration in the supernatant was measured.

(3) Results

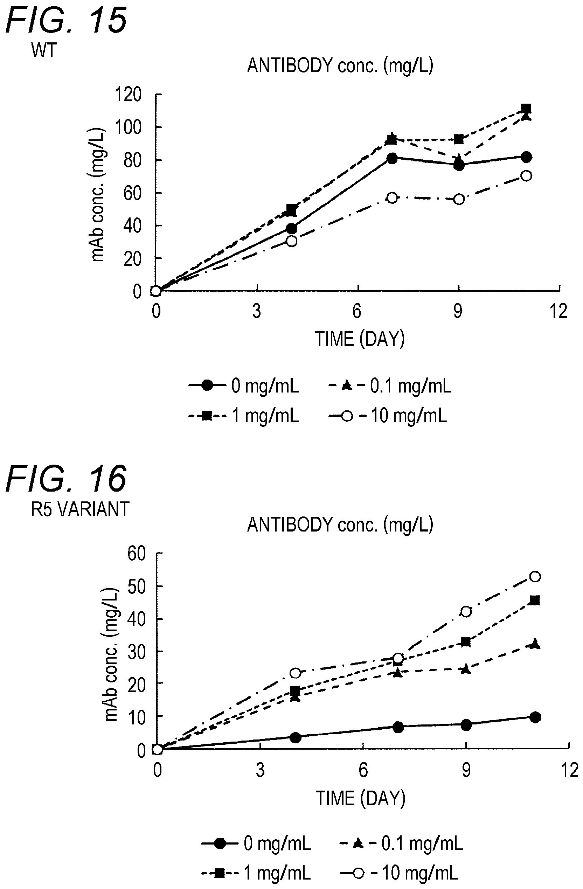

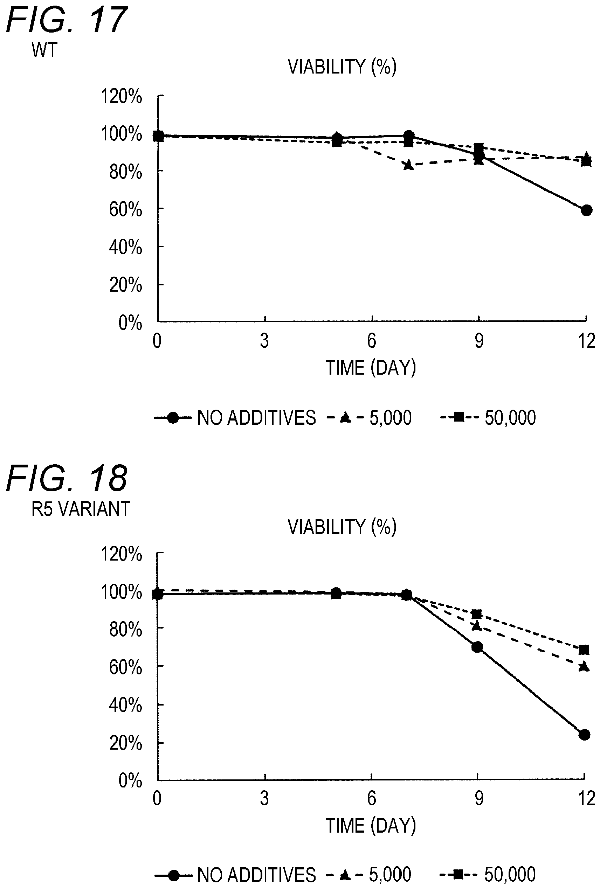

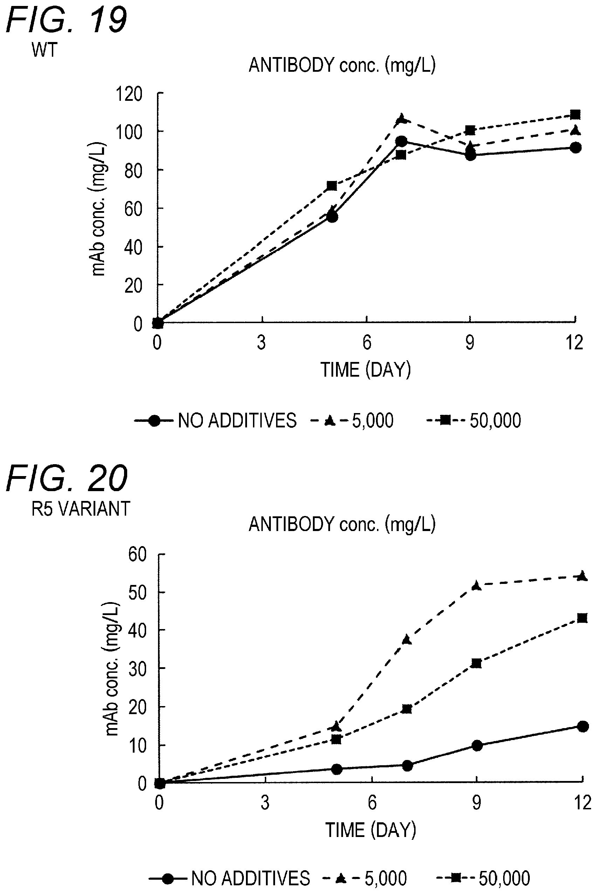

The viability (%) of each of the stable cell lines of rituximab and R5 variant in the test medium containing dextran sulfate sodium at different concentrations are shown in Figs. 13 and 14, and the antibody concentration (mg/L) in the supernatant is shown in Figs. 15 and 16. The viability (%) of each of the stable cell lines of rituximab and R5 variant in the test medium containing dextran sulfate sodium with different molecular weights is shown in Figs. 17 and 18, and the antibody concentration (mg/L) in the supernatant is shown in Figs. 19 and 20.

As shown in Figs. 13 and 14, the viability of each of the stable cell lines of rituximab and R5 variant on Day 11 tended to be higher as the concentration of dextran sulfate sodium was higher than that in the case where dextran sulfate sodium had not been added. As shown in Fig. 15, the yield of rituximab on Day 11 increased about 1.4 times when the concentration of dextran sulfate sodium in the medium was 0.1 or 1 mg/mL as compared to that in the case where dextran sulfate sodium had not been added. When the concentration was 10 mg/mL, the yield of rituximab was reduced as compared to that in the case where dextran sulfate sodium had not been added. As shown in Fig. 16, the yield of R5 variant on Day 11 was improved as the concentration of dextran sulfate sodium was higher, and the yield increased up to about 5.4 times as compared to that in the case where dextran sulfate sodium had not been added. Also, even at the lowest concentration of 0.1 mg/mL, the yield of R5 variant increased about 3.3 times as compared to that in the case where dextran sulfate sodium had not been added. Such an improvement in the yield of R5 variant was still a remarkable effect even in consideration of the improvement in viability by the addition of dextran sulfate sodium.

As shown in Figs. 17 and 18, in the case where dextran sulfate sodium with a molecular weight of 5,000 and dextran sulfate sodium with a molecular weight of 50,000 had been added, the viability of each of the stable cell lines of rituximab and R5 variant on Day 12 tended to be higher than that in the case where dextran sulfate sodium had not been added. As shown in Fig. 19, the yield of rituximab was slightly increased by the addition of dextran sulfate sodium as compared to that in the case where dextran sulfate sodium had not been added. As shown in Fig. 20, the yield of R5 variant on Day 12 in the case where dextran sulfate sodium had been added increased up to about 3.7 times as compared to that in the case where dextran sulfate sodium had not been added.

Example 3: Study of Molecular Weight of Dextran Sulfate Sodium (2)

The stable cell line of R3 variant of rituximab prepared in Example 1 was cultured in a medium containing dextran sulfate sodium of various molecular weights, and whether the yield of antibody was increased was studied.

(1) Culture of stable cell lines

A frozen stock of the stable cell line of R3 variant was thawed, cultured and passaged in the same manner as in Example 1. In the same manner as in Example 2, a test medium was prepared by adding dextran sulfate sodium (Merck KGaA) with a molecular weight of 5,000, dextran sulfate sodium (FUJIFILM Wako Pure Chemical Corporation) with a molecular weight of 50,000 or dextran sulfate sodium (FUJIFILM Wako Pure Chemical Corporation) with a molecular weight of 500,000 to a CD FortiCHO (trademark) medium containing 8 mM L-glutamine so as to have a concentration of 1 mg/mL. In the same manner as in Example 1, each stable cell line was seeded on each test medium, followed by culturing for 12 days. For comparison, each stable cell line was cultured for 12 days in a medium to which dextran sulfate sodium had not been added.

(2) Evaluation of survival and antibody production of stable cell lines

A part of the cell culture was collected as a specimen at the start of culture (Day 0) and on Days 5, 7, 9 and 12 from the start. In the same manner as in Example 1, the cell viability was calculated, and the antibody concentration in the supernatant was measured.

(3) Results

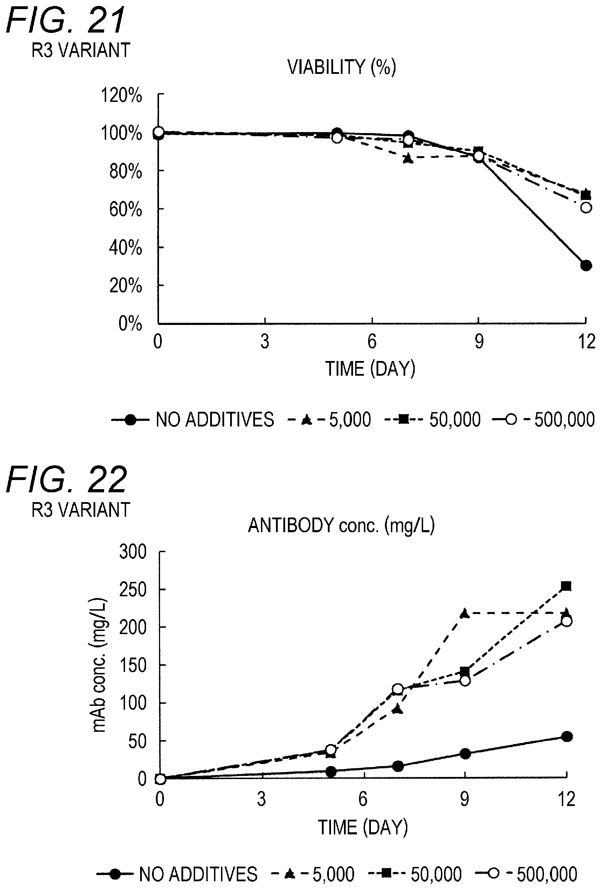

The viability (%) of the stable cell line of R3 variant is shown in Fig. 21, and the antibody concentration (mg/L) in the supernatant is shown in Fig. 22. As shown in Fig. 21, by the addition of dextran sulfate sodium, the viability of the stable cell line of R3 variant on Day 12 tended to be higher than that in the case where dextran sulfate sodium had not been added. As shown in Fig. 22, the yield of R3 variant on Day 12 was significantly increased by the addition of dextran sulfate sodium, and the yield increased up to about 4.6 times as compared to that in the case where dextran sulfate sodium had not been added. The difference in molecular weight of dextran sulfate sodium hardly affected the effect of improving the yield of R3 variant.

Example 4: Study of Concentration of Heparin Sodium

Each of the stable cell lines of rituximab and R5 variant prepared in Example 1 was cultured in a medium containing heparin sodium at various concentrations, and whether the yield of antibody was increased was studied.

(1) Culture of stable cell lines

Frozen stocks of each of the stable cell lines of rituximab and R5 variant were thawed, cultured, and passaged in the same manner as in Example 1. In the same manner as in Example 2, to a CD FortiCHO (trademark) medium containing 8 mM L-glutamine, heparin sodium (FUJIFILM Wako Pure Chemical Corporation) was added so as to have a concentration of 0.1, 1 or 10 mg/mL to prepare a test medium. In the same manner as in Example 1, each stable cell line was seeded on each test medium, followed by culturing for 12 days. For comparison, each stable cell line was cultured for 12 days in a medium to which heparin sodium had not been added.

(2) Evaluation of survival and antibody production of stable cell lines

A part of the cell culture was collected as a specimen at the start of culture (Day 0) and on Days 5, 7, 9 and 12 from the start. In the same manner as in Example 1, the cell viability was calculated, and the antibody concentration in the supernatant was measured.

(3) Results

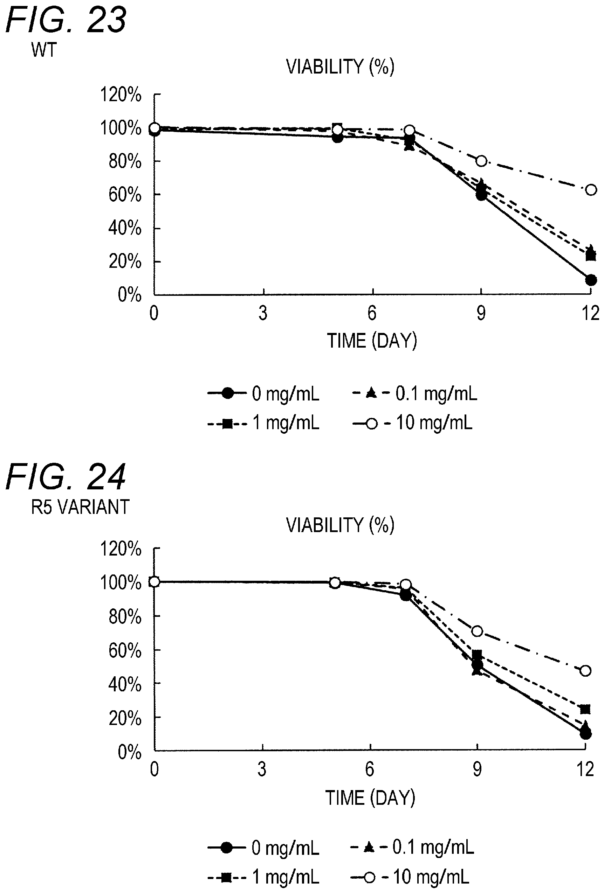

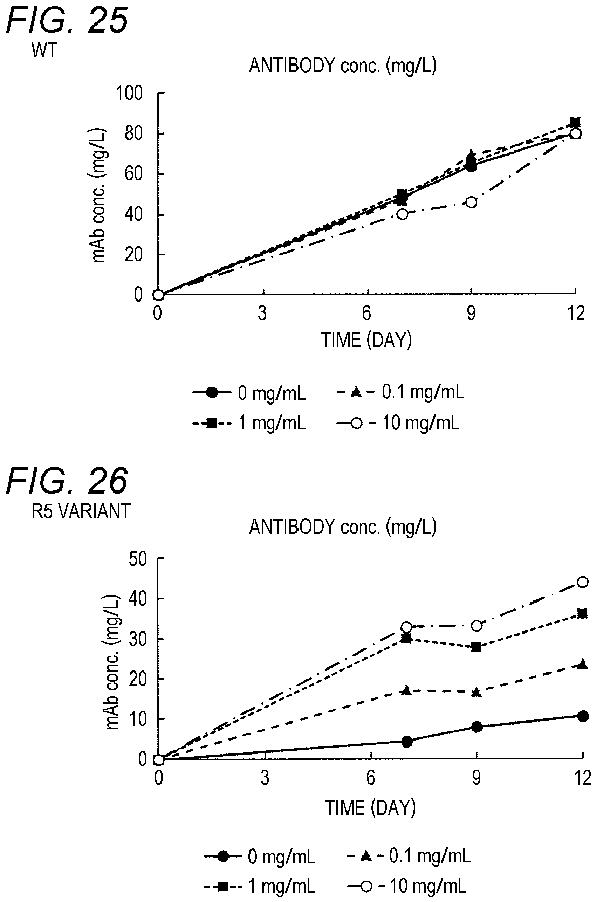

The viability (%) of each of the stable cell lines of rituximab and R5 variant is shown in Figs. 23 and 24, and the antibody concentration (mg/L) in the supernatant is shown in Figs. 25 and 26. As shown in Figs. 23 and 24, the viability of each of the stable cell lines on Day 12 was higher by the addition of heparin sodium as compared to the case where heparin sodium had not been added. In particular, when 10 mg/mL of heparin sodium had been added, the viability of each stable cell line on Day 12 was significantly higher than that in the case where heparin sodium had not been added.
As shown in Fig. 25, the yield of rituximab was almost the same as that in the case where heparin sodium had not been added even when added at any concentration. As shown in Fig. 26, the yield of R5 variant on Day 12 was improved as the concentration of heparin sodium was higher, and the yield increased up to about 4.2 times as compared to that in the case where heparin sodium had not been added. Also, even at the lowest concentration of 0.1 mg/mL, the yield of R5 variant increased about 2.2 times as compared to that in the case where heparin sodium had not been added. As described above, the viability of the stable cell line of rituximab was remarkably increased by addition of 10 mg/mL of heparin sodium, but there was no change in the yield of rituximab. Thus, it was suggested that the improvement in the yield of R5 variant is still a remarkable effect even in consideration of the improvement in viability by the addition of heparin sodium.

Example 5: Study on Effect of Anionic Polyamino Acid

An animal cell into which a gene encoding rituximab and its variant was introduced was cultured in a medium containing sodium polyaspartate or sodium polyglutamate as an anionic polyamino acid, and whether the yield of antibody was increased was studied.

(1) Culture of stable cell lines

Frozen stocks of each of the stable cell lines of rituximab, R3 variant and R5 variant were thawed, cultured, and passaged in the same manner as in Example 1. Sodium poly-$(\alpha,\beta)$-DL-aspartate (Merck KGaA) or sodium poly-L-$\gamma$-glutamate (Merck KGaA) was added to a CD FortiCHO (trademark) medium containing 8 mM L-glutamine so as to have a concentration of 1 mg/mL to prepare a test medium. In the same manner as in Example 1, each stable cell line was seeded on each test medium, followed by culturing for 12 days. For comparison, each stable cell line was cultured for 12 days in a medium to which an anionic polyamino acid had not been added.

(2) Evaluation of survival and antibody production of stable cell lines

A part of the cell culture was collected as a specimen at the start of culture (Day 0) and on Days 5, 7, 9 and 12 from the start. In the same manner as in Example 1, the cell viability was calculated, and the antibody concentration in the supernatant was measured.

(3) Results

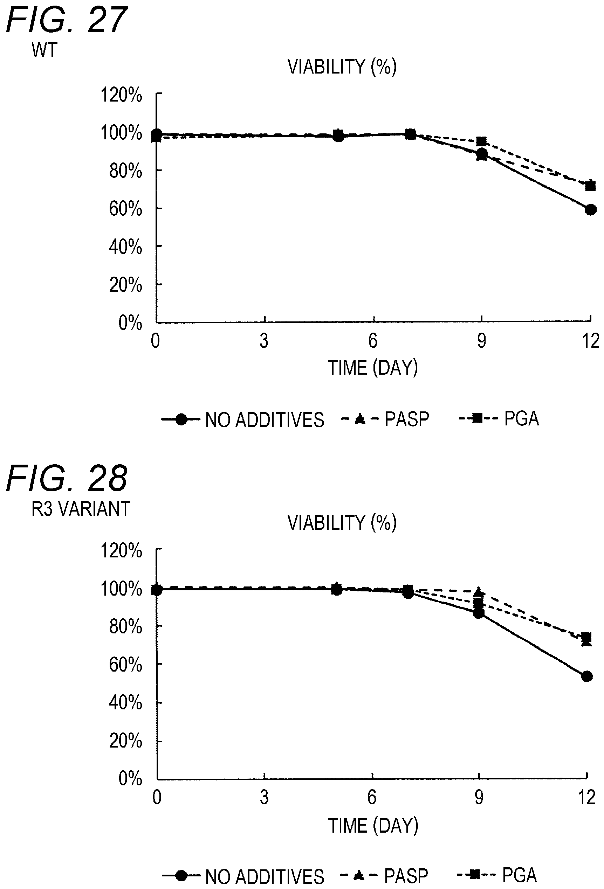

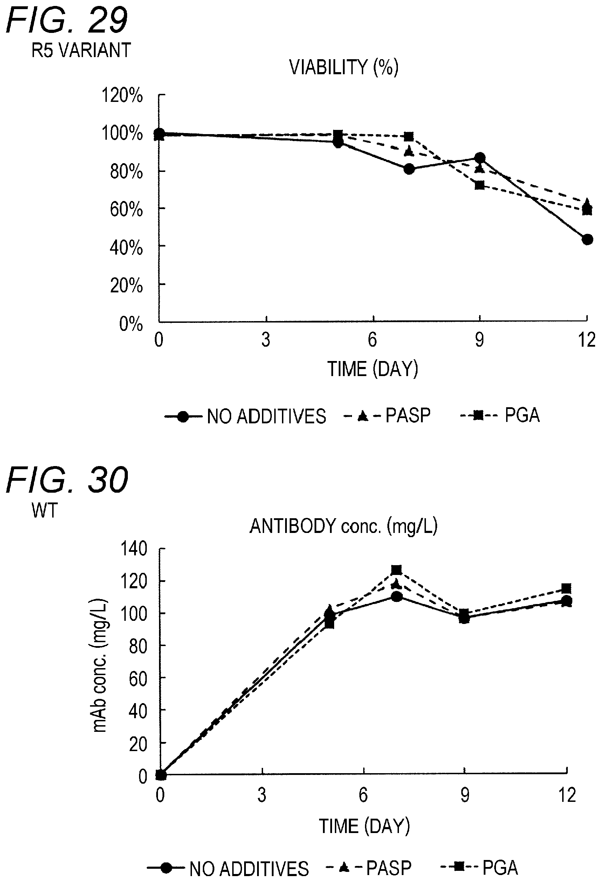

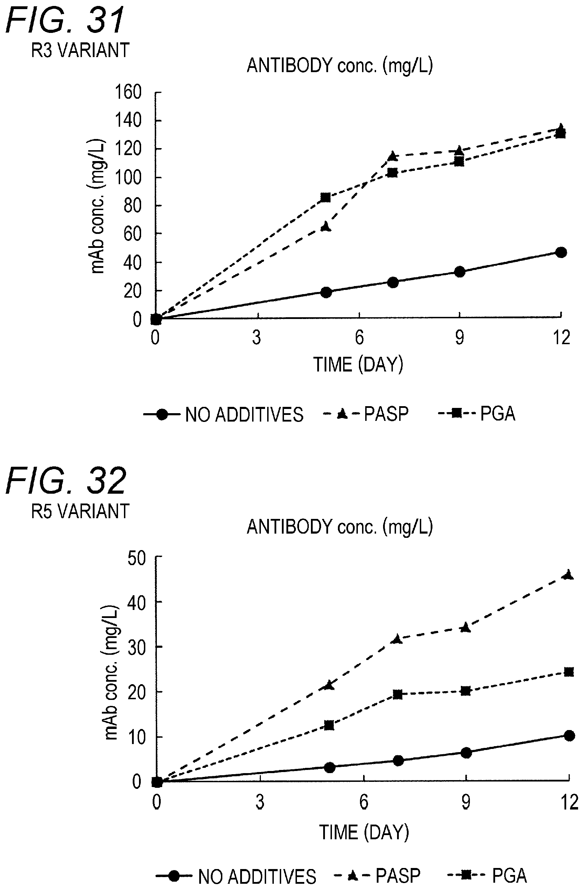

The viability (%) of each of the stable cell lines of rituximab, R3 variant and R5 variant are shown in Figs. 27 to 29, and the antibody concentration (mg/L) in the supernatant is shown in Figs. 30 to 32. In the figure, "PASP" refers to sodium polyaspartate, and "PGA" refers to sodium polyglutamate. As shown in Figs. 27 to 29, by the addition of anionic polyamino acid, the viability of each stable cell line on Day 12 tended to be higher than that in the case where the anionic polyamino acid had not been added. As shown in Fig. 30, the yield of rituximab was almost the same as that in the case where the anionic polyamino acid had not been added even when the anionic polyamino acid had been added. As shown in Fig. 31, the yield of R3 variant was remarkably increased by the addition of anionic polyamino acid as compared to that in the case where the anionic polyamino acid had not been added. As shown in Fig. 32, the yield of R5 variant was remarkably increased by the addition of anionic polyamino acid as compared to that in the case where the anionic polyamino acid had not been added. In particular, the yield of R5 variant on Day 12 in the case where sodium polyaspartate had been added increased about 4.5 times as compared to that in the case where anionic polyamino acid had not been added. From these results, it was shown that not only the sulfated polysaccharide but also the anionic polyamino acid can improve the yields of R3 and R5 variants of rituximab.

Example 6: Study of Effect of Neutral Polysaccharide

An animal cell into which a gene encoding rituximab and its variant was introduced was cultured in a medium containing dextran as a neutral polysaccharide, and whether the yield of antibody was increased was studied. For comparison, an experiment using a medium containing dextran sulfate sodium as an anionic polysaccharide was also performed.

(1) Culture of stable cell lines

Frozen stocks of each of the stable cell lines of rituximab, R3 variant and R5 variant were thawed, cultured, and passaged in the same manner as in Example 1. Dextran (molecular weight 5,000) (Sigma-Aldrich) or dextran sulfate sodium (molecular weight 5,000) (Sigma-Aldrich) was added to a CD FortiCHO (trademark) medium containing 8 mM L-glutamine so as to have a concentration of 1 mg/mL to prepare a test medium. In the same manner as in Example 1, each stable cell line was seeded on each test medium, followed by culturing for 12 days. For comparison, each stable cell line was cultured for 12 days in a medium to which any polysaccharide had not been added.

(2) Evaluation of survival and antibody production of stable cell lines

A part of the cell culture was collected as a specimen at the start of culture (Day 0) and on Days 2, 7 and 12 from the start. In the same manner as in Example 1, the cell viability was calculated, and the antibody concentration in the supernatant was measured.

(3) Results

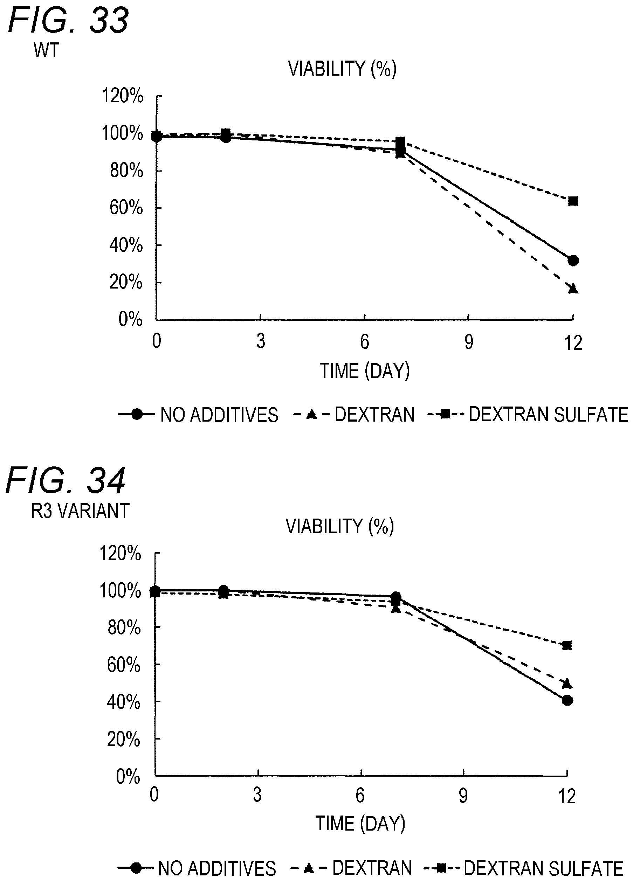

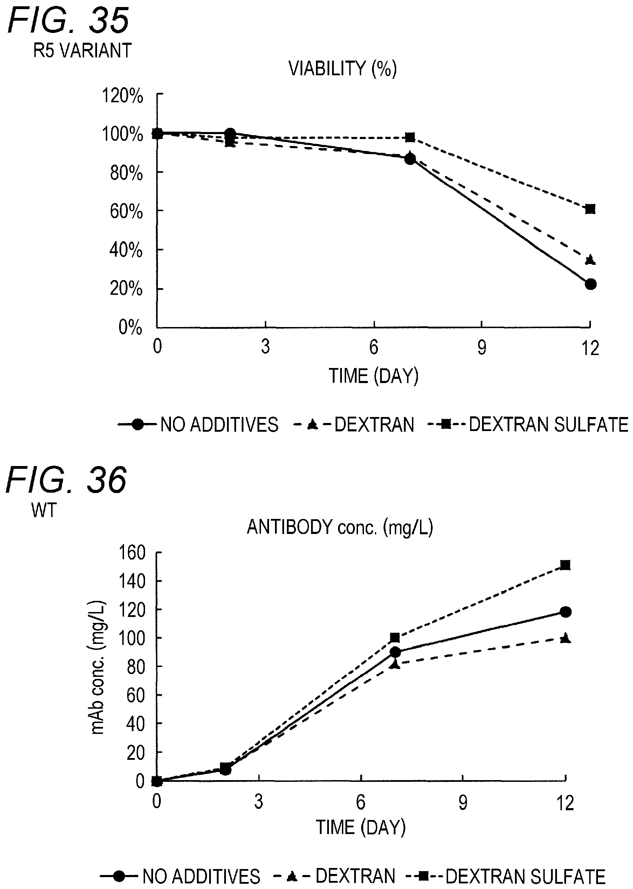

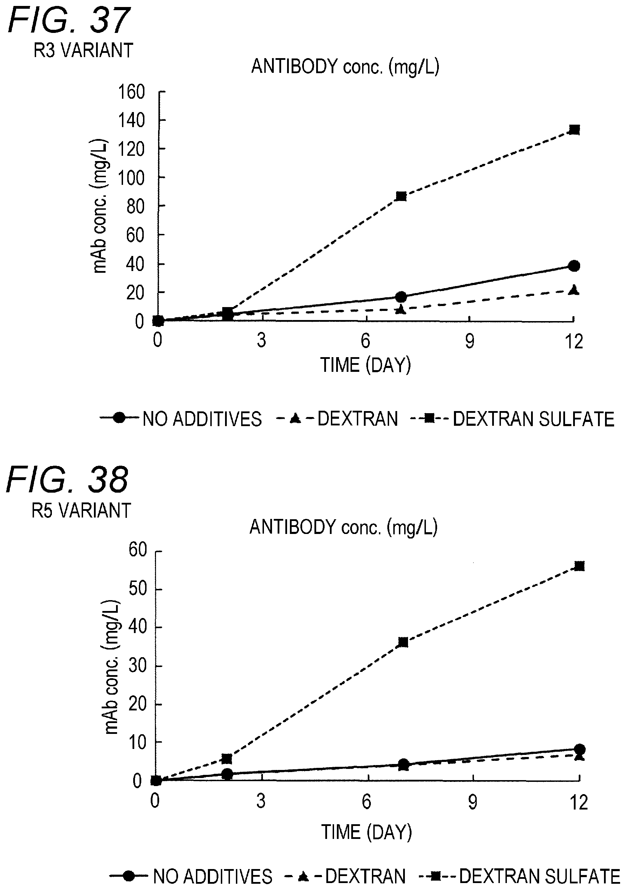

The viability (%) of each of the stable cell lines of rituximab, R3 variant and R5 variant are shown in Figs. 33 to 35, and the antibody concentration (mg/L) in the supernatant is shown in Figs. 36 to 38. As shown in Fig. 33, the viability of the stable cell line of rituximab on Day 12 was reduced by the addition of dextran as compared to that in the case where polysaccharide had not been added. As shown in Figs. 34 and 35, the viability of each of the stable cell lines of R3 variant and R5 variant on Day 12 was higher by the addition of dextran as compared to that in the case where polysaccharide had not been added. In all the stable cell lines, the viability on Day 12 was higher by the addition of dextran sulfate sodium as compared to that in the case where polysaccharide had not been added.
As shown in Figs. 36 to 38, the yields of rituximab, R3 variant, and R5 variant were all slightly reduced by the addition of dextran as compared to that in the case where polysaccharide had not been added. On the other hand, the yields of rituximab, R3 variant, and R5 variant were all increased by the addition of dextran sulfate sodium as compared to that in the case where polysaccharide had not been added. These results showed that dextran as a neutral polysaccharide does not affect the yields of rituximab, R3 variant and R5 variant.

Example 7: Study on Effect of Anionic Polysaccharide Without Sulfate Group and Neutral Polyamino Acid

An animal cell into which a gene encoding rituximab and R3 variant was introduced was cultured in a medium containing alginate as an anionic polysaccharide without a sulfate group or polyproline as a neutral polyamino acid, and whether the yield of antibody was increased was studied.

(1) Culture of stable cell lines

Frozen stocks of each of the stable cell lines of rituximab and R3 variant were thawed, cultured, and passaged in the same manner as in Example 1. Sodium alginate 80-120 (FUJIFILM Wako Pure Chemical Corporation) or poly-L-proline (Sigma-Aldrich) was added to a CD FortiCHO (trademark) medium containing 8 mM L-glutamine so as to have a concentration of 1 mg/mL to prepare a test medium. In the same manner as in Example 1, each stable cell line was seeded on each test medium, followed by culturing for 12 days. For comparison, each stable cell line was cultured for 12 days in a medium to which a polysaccharide and an anionic polyamino acid had not been added.

(2) Evaluation of survival and antibody production of stable cell lines

A part of the cell culture was collected as a specimen at the start of culture (Day 0) and on Days 5, 7, 9 and 12 from the start. In the same manner as in Example 1, the cell viability was calculated, and the antibody concentration in the supernatant was measured.

(3) Results

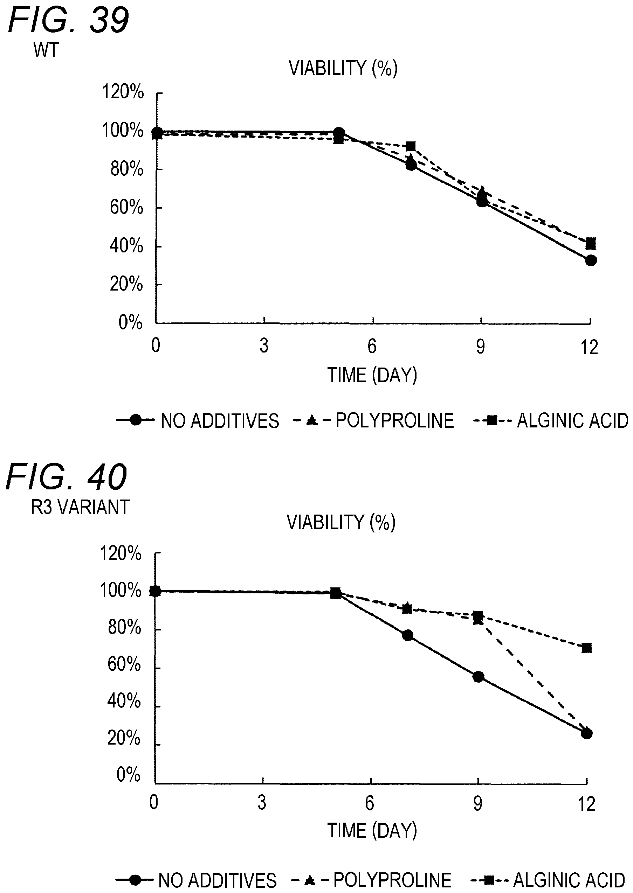

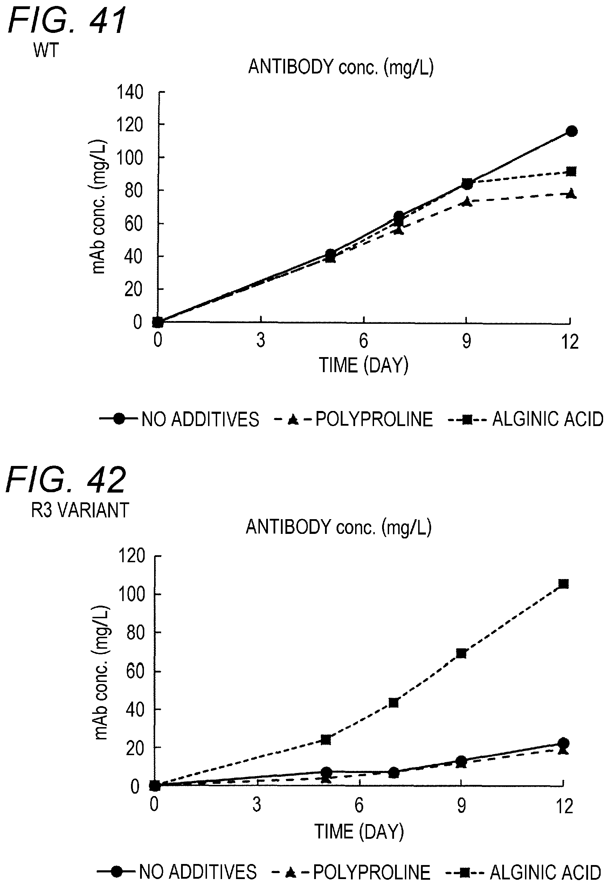

The viability (%) of each of the stable cell lines of rituximab and R3 variant is shown in Figs. 39 and 40, and the antibody concentration (mg/L) in the supernatant is shown in Figs. 41 and 42. As shown in Fig. 39, the viability of the stable cell line of rituximab on Day 12 was slightly higher by the addition of alginate or polyproline than that in the case where these had not been added. As shown in Fig. 40, the viability of the stable cell line of R3 variant on Day 9 was higher by the addition of alginate or polyproline than that in the case where these had not been added.
As shown in Fig. 41, the yield of rituximab was reduced by the addition of alginate or polyproline as compared to that in the case where these had not been added. As shown in Fig. 42, even when polyproline had been added, the yield of R3 variant was almost the same as that in the case where alginate and polyproline had not been added. On the other hand, the yield of R3 variant was significantly increased by the addition of alginate as compared to that in the case where alginate or polyproline had not been added. Such an improvement in the yield of R3 variant was still a remarkable effect even in consideration of the improvement in viability by the addition of alginate. From these results, it was shown that alginate, which is an anionic polysaccharide without a sulfate group, has an effect of improving the yield of rituximab and R3 variant, and polyproline as a neutral polyamino acid has no such effect.

Example 8: Study on Effect of Sulfated Polysaccharide in Production of Antibody Other Than Rituximab

An animal cell into which a gene encoding variants of various antibodies other than rituximab was introduced was cultured in a medium containing a sulfated polysaccharide, and whether the yield of antibody was increased was studied. In this example, a transient expression system was used. The affinity of each antibody for an antigen was also evaluated.

(1) Acquisition of genes of variants of various antibodies other than rituximab

In the same manner as the method described in U.S. Patent Application Publication No. 2018/0179298, a polynucleotide encoding a light chain of each antibody and its variant was synthesized by PCR using a plasmid DNA containing a gene encoding each of trastuzumab (humanized antibody), an anti-lysozyme antibody (mouse antibody), an anti-IL-6 antibody (mouse/human chimeric antibody) and an anti-IL-8 antibody (mouse/human chimeric antibody) as a template. In addition, a polynucleotide encoding the heavy chain of each antibody was synthesized. Variants of trastuzumab and the anti-IL-8 antibody were R3 variant and R4 variant. Variants of the anti-lysozyme antibody and the anti-IL-6 antibody were R5 variants.
The R3 variant of trastuzumab was an antibody in which amino acid residues at positions 65, 67 and 70 of the light chain defined by the Chothia method were substituted with arginine residues, and the R4 variant was an antibody in which amino acid residues at positions 63, 65, 67 and 70 of the light chain defined by the Chothia method were substituted with arginine residues. The R3 variant of the anti-IL-8 antibody was an antibody in which amino acid residues at positions 63, 65 and 67 of the light chain defined by the Chothia method were substituted with arginine residues, and the R4 variant was an antibody in which amino acid residues at positions 63, 65, 67 and 70 of the light chain defined by the Chothia method were substituted with arginine residues. The R5 variants of the anti-lysozyme antibody and the anti-IL-6 antibody were antibodies in which amino acid residues at positions 63, 65, 67, 70 and 72 of the light chain defined by the Chothia method were substituted with arginine residues.
A polynucleotide encoding the light chain of each antibody variant was inserted into pcDNA3.4 vector (Thermo Fisher Scientific Inc.). Thus, an animal cell expression plasmid DNA containing genes encoding the light chains of various antibodies and their variants (hereinafter, also referred to as "light chain plasmid") was obtained. In addition, a polynucleotide encoding the heavy chain of each antibody was inserted into the pcDNA3.4 vector to obtain an animal cell expression plasmid DNA containing a gene encoding the heavy chain of each antibody (hereinafter, also referred to as "heavy chain plasmid"). In order to evaluate the affinity for an antigen, using the animal cell expression plasmid DNA of rituximab and its R3 and R5 variants prepared in Example 1, light chain plasmids of rituximab and its R3 and R5 variants and a heavy chain plasmid of rituximab were also prepared.

Based on the base sequence of the gene encoding each antibody, amino acid sequences of a light chain and a heavy chain of the antibody (IgG) were determined. These amino acid sequences were as follows. Also, the amino acid sequences of the light chains of each prepared variant (IgG) are also shown below. The underlined portion indicates a position substituted with an arginine residue.

· Heavy chain of trastuzumab

EVQLVESGGGLVQPGGSLRLSCAASGFNIKDTYIHWVRQAPGKGLEWV

ARIYPTNGYTRYADSVKGRFTISADTSKNTAYLQMNSLRAEDTAVYYCSRWGGD

GFYAMDYWGQGTLVTVSSASTKGPSVFPLAPSSKSTSGGTAALGCLVKDYFPEP

VTVSWNSGALTSGVHTFPAVLQSSGLYSLSSVVTVPSSSLGTQTYICNVNHKPSN

TKVDKKVEPKSCDKTHTCPPCPAPELLGGPSVFLFPPKPKDTLMISRTPEVTCVVV

DVSHEDPEVKFNWYVDGVEVHNAKTKPREEQYNSTYRVVSVLTVLHQDWLNG

KEYKCKVSNKALPAPIEKTISKAKGQPREPQVYTLPPSRDELTKNQVSLTCLVKG

FYPSDIAVEWESNGQPENNYKTTPPVLDSDGSFFLYSKLTVDKSRWQQGNVFSCS

VMHEALHNHYTQKSLSLSPGK (SEQ ID NO: 5)

· Light chain of trastuzumab

DIQMTQSPSSLSASVGDRVTITCRASQDVNTAVAWYQQKPGKAPKLLIY

SASFLYSGVPSRFSGSRSGTDFTLTISSLQPEDFATYYCQQHYTTPPTFGQGTKVEI

KRTVAAPSVFIFPPSDEQLKSGTASVVCLLNNFYPREAKVQWKVDNALQSGNSQ

ESVTEQDSKDSTYSLSSTLTLSKADYEKHKVYACEVTHQGLSLPVTKSFNRGEC

(SEQ ID NO: 6)

· Light chain of R3 variant of trastuzumab

DIQMTQSPSSLSASVGDRVTITCRASQDVNTAVAWYQQKPGKAPKLLIY SASFLYSGVPSRFSGRRRGTRFTLTISSLQPEDFATYYCQQHYTTPPTFGQGTKVEI KRTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSLPVTKSFNRGEC (SEQ ID NO: 7)

· Light chain of R4 variant of trastuzumab

DIQMTQSPSSLSASVGDRVTITCRASQDVNTAVAWYQQKPGKAPKLLIY SASFLYSGVPSRFRGRRRGTRFTLTISSLQPEDFATYYCQQHYTTPPTFGQGTKVEI KRTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSLPVTKSFNRGEC (SEQ ID NO: 8)

· Heavy chain of anti-lysozyme antibody

DVQLQESGPSLVKPSQTLSLTCSVTGDSITSDYWSWIRKFPGNRLEYMGY VSYSGSTYYNPSLKSRISITRDTSKNQYYLDLNSVTTEDTATYYCANWDGDYWG QGTLVTVSAAKTTPPSVYPLAPGSAAQTNSMVTLGCLVKGYFPEPVTVTWNSGS LSSGVHTFPAVLQSDLYTLSSSVTVPSSTWPSETVTCNVAHPASSTKVDKKIVPRD CGCKPCICTVPEVSSVFIFPPKPKDVLTITLTPKVTCVVVDISKDDPEVQFSWFVDD VEVHTAQTQPREEQFNSTFRSVSELPIMHQDWLNGKEFKCRVNSAAFPAPIEKTIS KTKGRPKAPQVYTIPPPKEQMAKDKVSLTCMITDFFPEDITVEWQWNGQPAENY KNTQPIMDTDGSYFVYSKLNVQKSNWEAGNTFTCSVLHEGLHNHHTEKSLSHSP GK (SEQ ID NO: 9)

· Light chain of anti-lysozyme antibody

DIVLTQSPATLSVTPGNSVSLSCRASQSIGNNLHWYQQKSHESPRLLIKYA

SQSISGIPSRFSGSGSGTDFTLSINSVETEDFGMYFCQQSNSWPYTFGGGTKLEIKR

ADAAPTVSIFPPSSEQLTSGGASVVCFLNNFYPKDINVKWKIDGSERQNGVLNSW

TDQDSKDSTYSMSSTLTLTKDEYERHNSYTCEATHKTSTSPIVKSFNRNEC (SEQ

ID NO: 10)

· Light chain of R5 variant of anti-lysozyme antibody

DIVLTQSPATLSVTPGNSVSLSCRASQSIGNNLHWYQQKSHESPRLLIKYA

SQSISGIPSRF<u>R</u>G<u>R</u>G<u>R</u>GT<u>R</u>F<u>R</u>LSINSVETEDFGMYFCQQSNSWPYTFGGGTKLEIKR

ADAAPTVSIFPPSSEQLTSGGASVVCFLNNFYPKDINVKWKIDGSERQNGVLNSW

TDQDSKDSTYSMSSTLTLTKDEYERHNSYTCEATHKTSTSPIVKSFNRNEC (SEQ

ID NO: 11)

· Heavy chain of anti-IL-6 antibody

EVQLQQSGPELVKPGASVKMSCKASGYTFTSYVMHWVKQKPGQGLEWI

GYINPYNDGTKYNEKFKGKATLTSDKSSSTAYMELSSLTSEDSAVYYCAREGYG

NLERDCWGQGTSVTVSSASTKGPSVFPLAPSSKSTSGGTAALGCLVKDYFPEPVT

VSWNSGALTSGVHTFPAVLQSSGLYSLSSVVTVPSSSLGTQTYICNVNHKPSNTK

VDKKVEPKSCDKTHTCPPCPAPELLGGPSVFLFPPKPKDTLMISRTPEVTCVVVDV

SHEDPEVKFNWYVDGVEVHNAKTKPREEQYNSTYRVVSVLTVLHQDWLNGKE

YKCKVSNKALPAPIEKTISKAKGQPREPQVYTLPPSRDELTKNQVSLTCLVKGFYP

SDIAVEWESNGQPENNYKTTPPVLDSDGSFFLYSKLTVDKSRWQQGNVFSCSVM

HEALHNHYTQKSLSLSPGK (SEQ ID NO: 12)

· Light chain of anti-IL-6 antibody

DIVLTQSPASLAVSLGQRATISCRASESVDGFGISFMNWFQQKPGQPPKL
LIYVASNQGSGVPARFSGSGSGTDFSLNIHPMEEDDSAMYFCQQSKEVPWTFGGG
TKLEIKRTVAAPSVFIFPPSDEQLKSGTASVVCLLNNFYPREAKVQWKVDNALQS
GNSQESVTEQDSKDSTYSLSSTLTLSKADYEKHKVYACEVTHQGLSSPVTMSFNR
GEC (SEQ ID NO: 13)

· Light chain of R5 variant of anti-IL-6 antibody

DIVLTQSPASLAVSLGQRATISCRASESVDGFGISFMNWFQQKPGQPPKL
LIYVASNQGSGVPARFRGRGRGTRFRLNIHPMEEDDSAMYFCQQSKEVPWTFGG
GTKLEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTMSFN
RGEC (SEQ ID NO: 14)

· Heavy chain of anti-IL-8 antibody

EVKLVESGGGLVKPGGSLKLSCAASGFTFNNYAMSWVRQTPEKRLEWV
ASISSGGNTYYPDSVKGRFTLSRDNARNILYLQMSRLRSEDTAMYYCARDKLRLP
NWYFDVWGAGTTVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNVFSCS
VMHEALHNHYTQKSLSLSPGK (SEQ ID NO: 15)

· Light chain of anti-IL-8 antibody

DIVLTQSPPSLAVSLGQRATISCKASQSVDYDGDSYMNWYQQKPGQPPK

VLIYGASNLESGIPARFSGSGSGTDFTLNIYPVEEDAATYYCQQSNEDPPTFGGG

TKLEIKRTVAAPSVFIFPPSDEQLKSGTASVVCLLNNFYPREAKVQWKVDNALQS

GNSQESVTEQDSKDSTYSLSSTLTLSKADYEKHKVYACEVTHQGLSSPVTMSFNR

GEC (SEQ ID NO: 16)

· Light chain of R3 variant of anti-IL-8 antibody

DIVLTQSPPSLAVSLGQRATISCKASQSVDYDGDSYMNWYQQKPGQPPK

VLIYGASNLESGIPARF<u>R</u>GR<u>R</u>GTDFTLNIYPVEEDAATYYCQQSNEDPPTFGGG

TKLEIKRTVAAPSVFIFPPSDEQLKSGTASVVCLLNNFYPREAKVQWKVDNALQS

GNSQESVTEQDSKDSTYSLSSTLTLSKADYEKHKVYACEVTHQGLSSPVTMSFNR

GEC (SEQ ID NO: 17)

· Light chain of R4 variant of anti-IL-8 antibody

DIVLTQSPPSLAVSLGQRATISCKASQSVDYDGDSYMNWYQQKPGQPPK

VLIYGASNLESGIPARF<u>R</u>GR<u>R</u>GT<u>R</u>FTLNIYPVEEDAATYYCQQSNEDPPTFGGG

TKLEIKRTVAAPSVFIFPPSDEQLKSGTASVVCLLNNFYPREAKVQWKVDNALQS

GNSQESVTEQDSKDSTYSLSSTLTLSKADYEKHKVYACEVTHQGLSSPVTMSFNR

GEC (SEQ ID NO: 18)

(2) Introduction of gene encoding antibody into animal cell and cell culture

[Reagents]

Expi293 (trademark) Cells (Thermo Fisher Scientific Inc.)
Expi293 (trademark) Expression medium (Thermo Fisher Scientific Inc.)
ExpiFectamine (trademark) 293 transfection kit (Thermo Fisher Scientific Inc.)

(2.1) Transfection

Expi293 (trademark) Cells were proliferated by shaking culture (150 rpm) at 37°C in a 5% $CO_2$ atmosphere. 25.5 mL of cell culture ($3 \times 10^6$ cells/mL) was prepared according to the number of samples. A DNA solution with the following composition was prepared using a light chain plasmid and a heavy chain plasmid. The DNA solution was allowed to stand for 5 minutes.

[DNA Solution]

| | |
|---|---|
| Light chain plasmid solution | Amount ($\mu$L) corresponding to 15 $\mu$g |
| Heavy chain plasmid solution | Amount ($\mu$L) corresponding to 15 $\mu$g |
| Opti-MEM (trademark) | Appropriate amount (mL) |
| Total | 1.5 mL |

A transfection reagent having the following composition was prepared. The transfection reagent was allowed to stand for 5 minutes.

| | |
|---|---|
| ExpiFectamine reagent | 80 $\mu$L |
| Opti-MEM (trademark) | 1420 $\mu$L |
| Total | 1.5 mL |

The prepared DNA solution and the transfection reagent were mixed. The mixture was allowed to stand for 20 minutes. The resulting mixture (3 mL) was added to cell culture (25.5 mL) to perform transfection into cells. The transfected cells were shake-cultured (125 rpm) at 37°C for 4 hours in a 5% $CO_2$ atmosphere. Dextran sulfate sodium (molecular weight: 5000) (Sigma-Aldrich) was added to the cell culture so as to have a concentration of 1 mg/mL, and the mixture was further subjected to shaking culture. For comparison, the cell culture to which dextran sulfate sodium had not been added was similarly shaken and cultured. After 20 hours after transfection, 150 $\mu$L and 1.5 mL of ExpiFectamine (trademark) transfection enhancers 1 and 2 were added to the cell culture, respectively. Each mixture was shake-cultured (125 rpm) at 37°C for 4 days in a 5% $CO_2$ atmosphere. Culture supernatant was collected from each cell culture 5 days after transfection.

(3) Evaluation of antibody production

A part of the recovered culture supernatant was taken as a sample for measuring the antibody concentration. The concentration of each antibody in the culture supernatant was measured by sandwich ELISA method. Rituximab, trastuzumab, an anti-IL-6 antibody and an anti-IL-8 antibody were measured in the same manner as in Example 1. The anti-lysozyme antibody as a mouse antibody was measured as follows.

A solution of goat anti-mouse IgG (H+L) Highly Cross-Adsorbed (Thermo Fisher Scientific Inc.) diluted to 5 $\mu$g/mL with a KPL coating solution (SeraCare Life Sciences, Inc.) was added to each well of a 96-well plate (Corning Inc.), and the plate was incubated at 4°C overnight. After removing the solution from each well, 1% BSA/PBS was added, and the plate was incubated at room temperature for 3 hours. The solution was removed from each well, and then the plate washed three times with PBST. A culture supernatant was added to each well, and the plate was incubated at room temperature for 1 hour. The solution was removed from each well, and then the plate washed three times with PBST. A solution of HRP-labeled goat F(ab')2 fragment anti-mouse IgG (H+L) (Beckman Coulter, Inc.) diluted to 0.3 $\mu$g/mL with 1% BSA/PBS was added to each well, and the plate was incubated at room temperature for 1 hour. The solution was removed from each well, and then the plate washed three times with PBST. Chromogenic reaction was performed using KPL TMB (registered trademark) Peroxidase Substrate (SeraCare Life Sciences, Inc.) and the chromogenic reaction was stopped using KPL TMB (registered trademark) Peroxidase Stop Solution (SeraCare Life Sciences, Inc.). Absorbance at 450 nm was measured using a SpectraMax (registered trademark) 190 microplate reader (Molecular Devices, LLC). The concentration of the antibody in each supernatant was obtained using a standard curve of a reference antibody.

(4) Evaluation of affinity of modified antibody for antigen

(4.1) Purification of Antibody

An antibody in a culture supernatant recovered from the cell culture to which dextran sulfate sodium had been added was purified using MabSelect (trademark) (Cytiva). The resulting antibody solution was further purified by gel filtration chromatography using Superdex (registered trademark) 200 Increase 10/300 GL (Cytiva). PBS was used as a mobile phase, and purification was performed under the condition of a flow rate of 0.75 mL/min. The antibody concentration in the antibody solution obtained by purification was calculated by measuring the absorbance at 280 nm.

(4.2) Biotin labeling of trastuzumab and its variant

Trastuzumab and its R3 and R4 variants were labeled with biotin using Biotin Labeling Kit-NH$_2$ (DOJINDO LABORATORIES). Specific operations were carried out according to the package insert of the kit.

(4.3) Measurement of affinity for antigen

The affinity of each antibody for an antigen was measured by ELISA method. As antigens of each antibody, human CD20/MS4A1 full-length protein, His tag (HEK293) (ACROBiosystems Inc.), recombinant human ErbB2/Her2 Fc chimera (R&D Systems Inc.), chicken egg white-derived lysozyme (Sigma-Aldrich), animal free recombinant human IL-6 (PeproTech Inc.) and interleukin 8 (CXCL8) (Shenandoah Biotechnology, Inc.) were used. Specific operations are as follows. A solution of various antigens diluted to 5 μg/mL with PBS was added to each well of a 96-well plate (Nunc), and the plate was incubated at 4°C overnight. After removing the solution from each well, 1% BSA/PBS was added, and the plate was incubated at room temperature for 3 hours. The solution was removed from each well, and then the plate washed three times with PBST. An antibody solution was added to each well, and the plate was incubated at room temperature for 15 minutes. The solution was removed from each well, and then the plate washed three times with PBST. A solution of HRP-labeled anti-human IgG Fc antibody (Bethyl Laboratories, Inc.), HRP-labeled goat F(ab')2 fragment anti-mouse IgG (H+L) (Beckman Coulter, Inc.) or HRP-conjugated streptavidin (Thermo Fisher Scientific Inc.) diluted to 0.3 μg/mL with 1% BSA/PBS was added to each well, and the plate was incubated at room temperature for 1 hour. The solution was removed from each well, and then the plate washed three times with PBST. Chromogenic reaction was performed using KPL TMB (registered trademark) Peroxidase Substrate (SeraCare Life Sciences, Inc.) and the chromogenic reaction was stopped using KPL TMB (registered trademark) Peroxidase Stop Solution (SeraCare Life Sciences, Inc.). Absorbance at 450 nm was measured using a SpectraMax (registered trademark) 190 microplate reader (Molecular Devices, LLC).

(5) Results

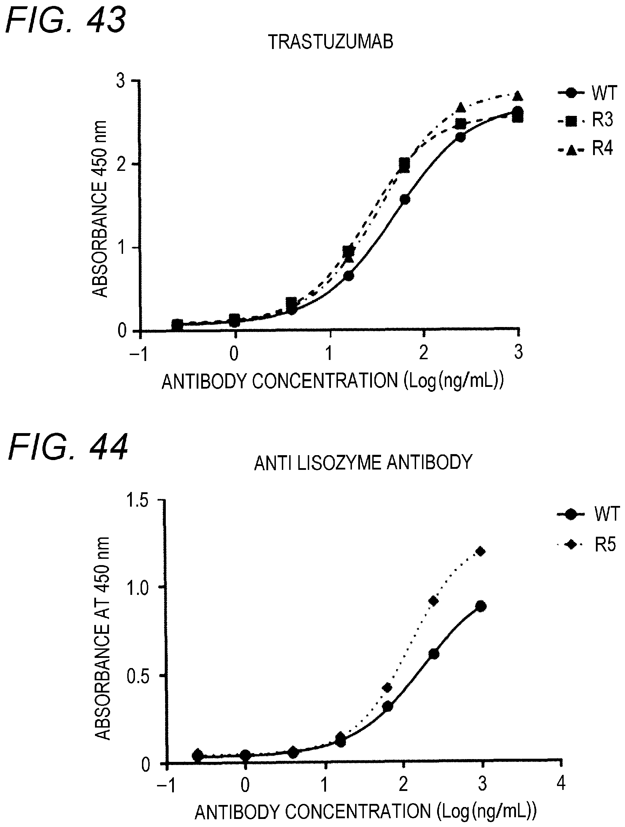

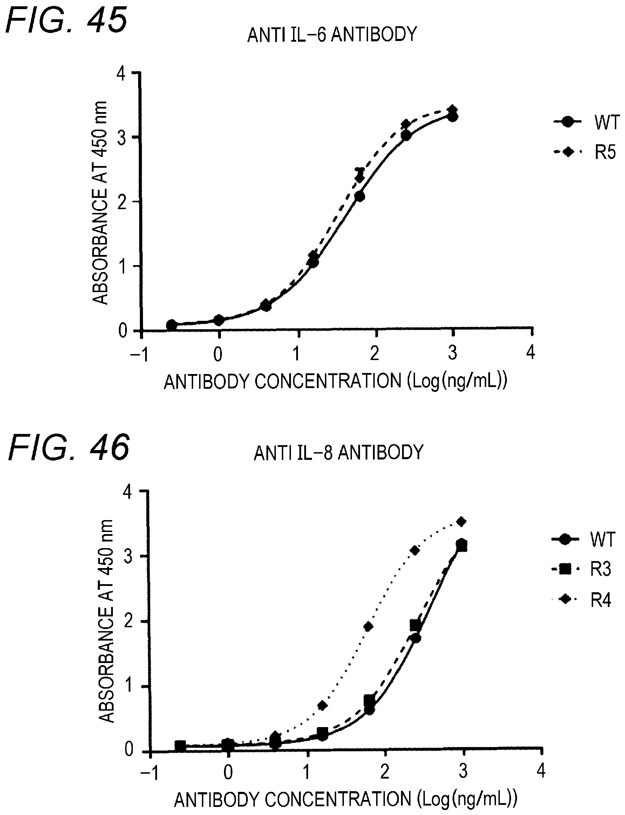

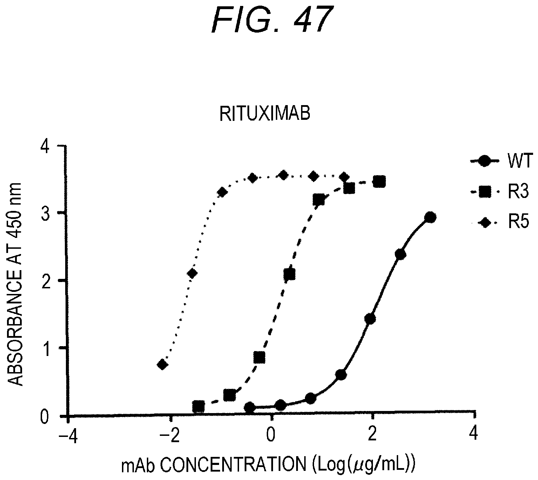

Expression levels and affinity for antigens of antibodies of other than rituximab and their variants are shown in Tables 1 to 4 and Figs. 43 to 46. Affinity of rituximab and its variants for antigens is shown in Table 5 and Fig. 47. In the tables and figures, "WT" refers to an antibody in which amino acid residues of the light chain FR3 are not substituted. In the table, "-DS" refers to an antibody concentration (mg/L) in the supernatant of the cell culture to which dextran sulfate sodium has not been added. "+DS" refers to an antibody concentration (mg/L) in the supernatant of the cell culture to which dextran sulfate sodium has been added. "Ratio" refers to a ratio of the value of +DS to the value of -DS. "EC50" refers to 50% effective concentration. "EC50 ratio" refers to a ratio of the affinity of each variant (EC50 ratio) when the EC50 in WT is taken as 1.0.

[Table 1]

| Trastuzumab | Expression level | | | Affinity | |
|---|---|---|---|---|---|
| | -DS (mg/L) | +DS (mg/L) | ratio | EC50 (ng/mL) | EC50 ratio |
| WT | 191.6 | 187.5 | 1.0 | 49.9 | 1.0 |
| R3 | 86.4 | 144.7 | 1.7 | 25.2 | 2.0 |
| R4 | 17.2 | 99.4 | 5.8 | 34.2 | 1.5 |

[Table 2]

| Anti-lysozyme antibody | Expression level | | | Affinity | |
|---|---|---|---|---|---|
| | -DS (mg/L) | +DS (mg/L) | ratio | EC50 (ng/mL) | EC50 ratio |
| WT | 992.0 | 896.9 | 0.9 | 179.0 | 1.0 |

24

(continued)

| Anti-lysozyme antibody | Expression level | | | Affinity | |
|---|---|---|---|---|---|
| | -DS (mg/L) | +DS (mg/L) | ratio | EC50 (ng/mL) | EC50 ratio |
| R5 | 28.5 | 134.0 | 4.7 | 124.2 | 1.4 |

[Table 3]

| Anti-IL-6 antibody | Expression level | | | Affinity | |
|---|---|---|---|---|---|
| | -DS (mg/L) | +DS (mg/L) | ratio | EC50 (ng/mL) | EC50 ratio |
| WT | 25.6 | 23.4 | 0.9 | 41.0 | 1.0 |
| R5 | 11.1 | 20.5 | 1.9 | 32.7 | 1.3 |

[Table 4]

| Anti-IL-8 antibody | Expression level | | | Affinity | |
|---|---|---|---|---|---|
| | -DS (mg/L) | +DS (mg/L) | ratio | EC50 (ng/mL) EC50 ratio | |
| WT | 36.6 | 35.6 | 1.0 | 389.8 | 1.0 |
| R3 | 17.9 | 24.7 | 1.4 | 269.2 | 1.4 |
| R4 | 8.9 | 20.5 | 2.3 | 59.8 | 6.5 |

[Table 5]

| Rituximab | Affinity | |
|---|---|---|
| | EC50 ($\mu$g/mL) | EC50 ratio |
| WT | 127.3 | 1.0 |
| R3 | 1.707 | 74.6 |
| R5 | 0.027 | 4707.8 |

As shown in Tables 1 to 4, the yields of trastuzumab, anti-lysozyme antibody, anti-IL-6 antibody and anti-IL-8 antibody were almost the same even when dextran sulfate sodium had been added as compared to those in the case where dextran sulfate sodium had not been added. On the other hand, the yields of variants of antibodies were clearly increased by the addition of dextran sulfate sodium as compared to those in the case where dextran sulfate sodium had not been added. In the variants of the antibodies, while it is common that a predetermined amino acid residue of the light chain FR3 is substituted with an arginine residue, the amino acid sequences of the light chain and the heavy chain are different from each other. Therefore, it was suggested that the effect of increasing the yields of variants by the polyanionic compound can be applied to variants of any antibody. In addition, as shown in Tables 1 to 5 and Figs. 43 to 47, the variants of all antibodies also had improved affinity for antigens as compared with WT. It was suggested that the addition of dextran sulfate sodium to the medium does not affect the affinity of the antibody for an antigen.

**Claims**

1. A method for producing an antibody, comprising culturing in a presence of a polyanionic compound an animal cell into which a gene encoding an antibody has been introduced whereby the animal cell produces the antibody,

wherein the polyanionic compound is at least one selected from the group consisting of an anionic polysaccharide and an anionic polyamino acid, and

the antibody is an antibody in which at least 3 amino acid residues of framework region 3 (FR3) comprise or have been substituted each independently with an arginine residue or a lysine residue.

2. The method according to claim 1, wherein the anionic polysaccharide is a sulfated polysaccharide or alginate.

3. The method according to claim 2, wherein the sulfated polysaccharide is at least one selected from the group consisting of dextran sulfate , a salt thereof, glycosaminoglycan, and proteoglycan comprising a sulfate group.

4. The method according to claim 3, wherein the glycosaminoglycan is at least one selected from the group consisting of heparin, heparan sulfate, chondroitin sulfate, dermatan sulfate, keratan sulfate, pentosan sulfate, and salts thereof.

5. The method according to claim 3 or 4, wherein the proteoglycan comprising a sulfate group is a protein to which at least one selected from the group consisting of heparin, heparan sulfate, chondroitin sulfate, dermatan sulfate, keratan sulfate, and salts thereof is covalently bonded.

6. The method according to any one of claims 1 to 5, wherein the anionic polyamino acid is at least one selected from the group consisting of polyglutamic acid, polyaspartic acid, and salts thereof.

7. The method according to any one of claims 1 to 6, wherein the animal cell is cultured in a medium comprising the polyanionic compound at a concentration of 0.05 mg/mL or more and 20 mg/mL or less.

8. The method according to any one of claims 1 to 7, wherein the antibody is IgG.

9. The method according to any one of claims 1 to 8, wherein the at least 3 amino acid residues of the FR3 comprise residues in at least three positions selected from the group consisting of positions 63, 65, 67, 70 and 72 of a light chain defined by Chothia method.

10. The method according to any one of claims 1 to 9, wherein the antibody is an antibody in which amino acid residues at positions 63, 65 and 67 comprise or have been substituted each independently with an arginine residue or a lysine residue.

11. The method according to any one of claims 1 to 10, wherein the antibody is an antibody in which amino acid residues at positions 63, 65, 67 and 70 comprise or have been substituted each independently with an arginine residue or a lysine residue.

12. The method according to any one of claims 1 to 11, wherein the antibody is an antibody in which amino acid residues at positions 63, 65, 67, 70 and 72 comprise or have been substituted each independently with an arginine residue or a lysine residue.

13. The method according to any one of claims 1 to 12, wherein the antibody is an antibody in which at least 3 amino acid residues of framework region 3 (FR3) have been substituted each independently with an arginine residue or a lysine residue and which has improved affinity for an antigen as compared with said antibody before substitution of the amino acid residues.

## FIG. 1
WT

## FIG. 2
R3 VARIANT

# FIG. 3

R5 VARIANT

VIABILITY (%)

NO ADDITIVES   - ▲ - GLUCOSE
-■-- SUCROSE    -○- TREHALOSE

# FIG. 4

WT

VIABILITY (%)

NO ADDITIVES        - ▲ - CHONDROITIN SULFATE
-■-- DEXTRAN SULFATE  -○- HEPARIN

## FIG. 5

R3 VARIANT

## FIG. 6

R5 VARIANT

## FIG. 7

WT

ANTIBODY conc. (mg/L)

NO ADDITIVES  - ▲ - GLUCOSE
- ■ - SUCROSE  —○ - TREHALOSE

## FIG. 8

R3 VARIANT

ANTIBODY conc. (mg/L)

NO ADDITIVES  - ▲ - GLUCOSE
- ■ - SUCROSE  —○ - TREHALOSE

## FIG. 9

R5 VARIANT

ANTIBODY conc. (mg/L)

NO ADDITIVES — GLUCOSE
SUCROSE — TREHALOSE

## FIG. 10

WT

ANTIBODY conc. (mg/L)

NO ADDITIVES — CHONDROITIN SULFATE
DEXTRAN SULFATE — HEPARIN

## FIG. 11

R3 VARIANT

ANTIBODY conc. (mg/L)

- NO ADDITIVES    - ▲ - CHONDROITIN SULFATE
- ■ - DEXTRAN SULFATE    - O - HEPARIN

## FIG. 12

R5 VARIANT

ANTIBODY conc. (mg/L)

- NO ADDITIVES    - ▲ - CHONDROITIN SULFATE
- ■ - DEXTRAN SULFATE    - O - HEPARIN

*FIG. 13*
WT

VIABILITY (%)

TIME (DAY)

● 0 mg/mL ▲ 0.1 mg/mL
■ 1 mg/mL ○ 10 mg/mL

*FIG. 14*
R5 VARIANT

VIABILITY (%)

TIME (DAY)

● 0 mg/mL ▲ 0.1 mg/mL
■ 1 mg/mL ○ 10 mg/mL

## FIG. 15
WT

ANTIBODY conc. (mg/L)

- ● — 0 mg/mL
- ▲ - 0.1 mg/mL
- ■-- 1 mg/mL
- ○ - 10 mg/mL

## FIG. 16
R5 VARIANT

ANTIBODY conc. (mg/L)

- ● — 0 mg/mL
- ▲ - 0.1 mg/mL
- ■-- 1 mg/mL
- ○ - 10 mg/mL

## FIG. 17
WT

## FIG. 18
R5 VARIANT

## FIG. 19
WT

ANTIBODY conc. (mg/L)

NO ADDITIVES   — ▲ — 5,000   — ■ — 50,000

## FIG. 20
R5 VARIANT

ANTIBODY conc. (mg/L)

NO ADDITIVES   — ▲ — 5,000   — ■ — 50,000

## FIG. 21
R3 VARIANT

## FIG. 23
WT

VIABILITY (%)

```
120%
100%
 80%
 60%
 40%
 20%
  0%
     0      3      6      9     12
```

TIME (DAY)

● — 0 mg/mL    - ▲ - 0.1 mg/mL

-■-- 1 mg/mL    —○ - 10 mg/mL

## FIG. 24
R5 VARIANT

VIABILITY (%)

```
120%
100%
 80%
 60%
 40%
 20%
  0%
     0      3      6      9     12
```

TIME (DAY)

● — 0 mg/mL    - ▲ - 0.1 mg/mL

-■-- 1 mg/mL    —○ - 10 mg/mL

## FIG. 25

WT

ANTIBODY conc. (mg/L)

- ●─ 0 mg/mL
- ▲ - 0.1 mg/mL
- ■-- 1 mg/mL
- ○ - 10 mg/mL

## FIG. 26

R5 VARIANT

ANTIBODY conc. (mg/L)

- ●─ 0 mg/mL
- ▲ - 0.1 mg/mL
- ■-- 1 mg/mL
- ○ - 10 mg/mL

## *FIG. 27*
WT

VIABILITY (%)

## *FIG. 28*
R3 VARIANT

VIABILITY (%)

## FIG. 29
R5 VARIANT

VIABILITY (%)

—●— NO ADDITIVES  – ▲ – PASP  – ■ – PGA

## FIG. 30
WT

ANTIBODY conc. (mg/L)

—●— NO ADDITIVES  – ▲ – PASP  – ■ – PGA

## FIG. 31
R3 VARIANT

ANTIBODY conc. (mg/L)

## FIG. 32
R5 VARIANT

ANTIBODY conc. (mg/L)

## FIG. 33
WT

VIABILITY (%)

TIME (DAY)

—●— NO ADDITIVES  - ▲ - DEXTRAN  -■-- DEXTRAN SULFATE

## FIG. 34
R3 VARIANT

VIABILITY (%)

TIME (DAY)

—●— NO ADDITIVES  - ▲ - DEXTRAN  -■-- DEXTRAN SULFATE

## FIG. 35
R5 VARIANT

VIABILITY (%)

NO ADDITIVES  - ▲ -DEXTRAN  - ■-- DEXTRAN SULFATE

## FIG. 36
WT

ANTIBODY conc. (mg/L)

NO ADDITIVES  - ▲ - DEXTRAN  -■-- DEXTRAN SULFATE

## FIG. 37

R3 VARIANT

ANTIBODY conc. (mg/L)

─●─NO ADDITIVES  ─▲─DEXTRAN  ─■─DEXTRAN SULFATE

## FIG. 38

R5 VARIANT

ANTIBODY conc. (mg/L)

─●─NO ADDITIVES  ─▲─DEXTRAN  ─■─DEXTRAN SULFATE

# FIG. 39
WT

VIABILITY (%)

─●─ NO ADDITIVES  ─▲─ POLYPROLINE  ──■── ALGINIC ACID

# FIG. 40
R3 VARIANT

VIABILITY (%)

─●─ NO ADDITIVES  ─▲─ POLYPROLINE  ──■── ALGINIC ACID

## FIG. 41
WT

ANTIBODY conc. (mg/L)

—●— NO ADDITIVES  - ▲ - POLYPROLINE  --■-- ALGINIC ACID

## FIG. 42
R3 VARIANT

ANTIBODY conc. (mg/L)

—●— NO ADDITIVES  - ▲ - POLYPROLINE  --■-- ALGINIC ACID

*FIG. 43*

TRASTUZUMAB

*FIG. 44*

ANTI LISOZYME ANTIBODY

## FIG. 45

### ANTI IL-6 ANTIBODY

## FIG. 46

### ANTI IL-8 ANTIBODY

# FIG. 47

RITUXIMAB

**EUROPEAN SEARCH REPORT**

Application Number

EP 22 20 5436

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MELLAHI KAHINA ET AL: "Process intensification for the production of rituximab by an inducible CHO cell line", BIOPROCESS AND BIOSYSTEMS ENGINEERING, SPRINGER, DE, vol. 42, no. 5, 23 January 2019 (2019-01-23), pages 711-725, XP036768703, ISSN: 1615-7591, DOI: 10.1007/S00449-019-02075-Z [retrieved on 2019-01-23] * abstract * * page 712, right-hand column, paragraph 2 * | 1-13 | INV. C07K16/24 C07K16/28 C07K16/32 C07K16/40 |
| Y | US 2014/274911 A1 (COLLINS BRIAN EDWARD [US] ET AL) 18 September 2014 (2014-09-18) * paragraph [0003] * * paragraph [0006] * * paragraph [0066] * * paragraph [0067] * | 1-13 | |
| | | | TECHNICAL FIELDS SEARCHED (IPC) |
| Y | HYOUNG PARK JIN ET AL: "The molecular weight and concentration of dextran sulfate affect cell growth and antibody production in CHO cell cultures", BIOTECHNOLOGY PROGRESS, vol. 32, no. 5, 1 September 2016 (2016-09-01), pages 1113-1122, XP055899953, ISSN: 8756-7938, DOI: 10.1002/btpr.2287 * abstract * * page 1114, left-hand column, paragraph 3 * | 1-13 | C07K |

-/--

The present search report has been drawn up for all claims

| Place of search | Date of completion of the search | Examiner |
|---|---|---|
| Munich | 10 March 2023 | Malamoussi, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

**EUROPEAN SEARCH REPORT**

Application Number

EP 22 20 5436

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LI LING ET AL: "Heparin Promotes Suspension Adaptation Process of CHO-TS28 Cells by Eliminating Cell Aggregation", MOLECULAR BIOTECHNOLOGY, vol. 47, no. 1, 30 June 2010 (2010-06-30), pages 9-17, XP093029989, New York ISSN: 1073-6085, DOI: 10.1007/s12033-010-9306-1 Retrieved from the Internet: URL:http://link.springer.com/article/10.10 07/s12033-010-9306-1/fulltext.html> * abstract * ----- | 1-13 | |
| Y | FUKUNAGA ATSUSHI ET AL: "Improvement of antibody affinity by introduction of basic amino acid residues into the framework region", BIOCHEMISTRY AND BIOPHYSICS REPORTS, vol. 15, 1 September 2018 (2018-09-01), pages 81-85, XP055828390, ISSN: 2405-5808, DOI: 10.1016/j.bbrep.2018.07.005 * abstract * * page 82, left-hand column, paragraph 1 * * figure 1 * * paragraph [0082] * ----- | 1-13 | TECHNICAL FIELDS SEARCHED (IPC) |
| Y | EP 3 342 783 A1 (SYSMEX CORP [JP]) 4 July 2018 (2018-07-04) * paragraph [0129] * * paragraph [0099] * * paragraph [0109] * * table 3 * ----- | 1-13 | |
| Y | WO 2020/257760 A1 (SINGLE CELL TECH INC [US]) 24 December 2020 (2020-12-24) * sequence 465 * ----- | 1-13 | |

-/--

The present search report has been drawn up for all claims

| Place of search | Date of completion of the search | Examiner |
|---|---|---|
| Munich | 10 March 2023 | Malamoussi, A |

EPO FORM 1503 03.82 (P04C01)

page 2 of 3

Europäisches
Patentamt

European
Patent Office

Office européen
des brevets

**EUROPEAN SEARCH REPORT**

Application Number

EP 22 20 5436

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2013/131001 A1 (ACADEMIA SINICA; LIANG CHI MING [US]) 6 September 2013 (2013-09-06) * sequence 16 * ----- | 1-13 | |
| | | | **TECHNICAL FIELDS SEARCHED (IPC)** |

The present search report has been drawn up for all claims

| Place of search | Date of completion of the search | Examiner |
|---|---|---|
| Munich | 10 March 2023 | Malamoussi, A |

EPO FORM 1503 03.82 (P04C01)

page 3 of 3

## ANNEX TO THE EUROPEAN SEARCH REPORT
## ON EUROPEAN PATENT APPLICATION NO.

EP 22 20 5436

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2023

| Patent document cited in search report | | Publication date | Patent family member(s) | | Publication date |
|---|---|---|---|---|---|
| US 2014274911 | A1 | 18-09-2014 | CN | 105189761 A | 23-12-2015 |
| | | | EP | 2971014 A1 | 20-01-2016 |
| | | | US | 2014274911 A1 | 18-09-2014 |
| | | | US | 2017233782 A1 | 17-08-2017 |
| | | | WO | 2014159488 A1 | 02-10-2014 |
| EP 3342783 | A1 | 04-07-2018 | CN | 108250294 A | 06-07-2018 |
| | | | EP | 3342783 A1 | 04-07-2018 |
| | | | US | 2018179298 A1 | 28-06-2018 |
| | | | US | 2022235147 A1 | 28-07-2022 |
| WO 2020257760 | A1 | 24-12-2020 | AU | 2020298324 A1 | 27-01-2022 |
| | | | CA | 3143995 A1 | 24-12-2020 |
| | | | CN | 114729040 A | 08-07-2022 |
| | | | EP | 3986936 A1 | 27-04-2022 |
| | | | IL | 289112 A | 01-02-2022 |
| | | | JP | 2022537053 A | 23-08-2022 |
| | | | US | 2022332816 A1 | 20-10-2022 |
| | | | WO | 2020257760 A1 | 24-12-2020 |
| WO 2013131001 | A1 | 06-09-2013 | CN | 104168916 A | 26-11-2014 |
| | | | EP | 2819695 A1 | 07-01-2015 |
| | | | JP | 6163502 B2 | 12-07-2017 |
| | | | JP | 2015517982 A | 25-06-2015 |
| | | | TW | 201350507 A | 16-12-2013 |
| | | | US | 2015017230 A1 | 15-01-2015 |
| | | | WO | 2013131001 A1 | 06-09-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180179298

**Non-patent literature cited in the description**

- **CHOTHIA C. ; LESK AM.** Canonical Structures for the Hypervariable Regions of Immunoglobulins. *J Mol Biol.,* 1987, vol. 196, 901-917

- **KOHLER ; MILSTEIN.** *Nature,* 1975, vol. 256, 495-497